# EUROPEAN PATENT SPECIFICATION

(11) **EP 4 549 586 B1**
(45) Date of publication and mention of the grant of the patent: **09.12.2026**
(21) Application number: 25162154.6
(22) Date of filing: 20.03.2018
(51) Int. Cl.: C12Q 1/6883

(54) **TREATMENT AND INHIBITION OF INFLAMMATORY LUNG DISEASES IN PATIENTS HAVING RISK ALLELES IN THE GENES ENCODING IL33 AND IL1RL1**
BEHANDLUNG UND HEMMUNG VON ENTZÜNDLICHEN LUNGENERKRANKUNGEN BEI PATIENTEN MIT RISIKOALLELEN IN DEN FÜR IL33 UND IL1RL1 CODIERENDEN GENEN
TRAITEMENT ET INHIBITION DES MALADIES PULMONAIRES INFLAMMATOIRES CHEZ DES PATIENTS POSSÉDANT DES ALLÈLES DE RISQUE DANS LES GÈNES CODANT POUR L'IL33 ET IL1RL1

(30) Priority: 13.04.2017 US 201762485077 P
(43) Date of publication of application: 07.05.2025
(60) Divisional application: 26215205.1
(62) Document number(s) of the earlier application(s) in accordance with Art. 76 EPC: 21162316.0 / 3 957 752; 18716446.2 / 3 610 041
(73) Proprietor: Regeneron Pharmaceuticals, Inc., Tarrytown, NY 10591 (US)
(72) Inventor: Bruse, Shannon, Tarrytown, NY 10591 (US); Mccarthy, Shane, Tarrytown, NY 10591 (US); Baras, Aris, Tarrytown, NY 10591 (US); Dewey, Frederick, Tarrytown, NY 10591 (US); Gottesman, Omri, Tarrytown, NY 10591 (US)
(74) Representative: CH Kilger Anwaltspartnerschaft mbB

(56) References cited:
- US-A1- 2010 160 802
- US-A1- 2014 271 642
- US-A1- 2016 168 640
- ASIF S TULAH ET AL: "Defining the contribution of SNPs identified in asthma GWAS to clinical variables in asthmatic children", BMC MEDICAL GENETICS, BIOMED CENTRAL, LONDON, GB, vol. 14, no. 1, 25 September 2013 (2013-09-25), pages 100, XP021163432, ISSN: 1471-2350, DOI: 10.1186/1471-2350-14-100
- NÉOMI S. GROTENBOER ET AL: "Decoding asthma: Translating genetic variation in IL33 and IL1RL1 into disease pathophysiology", JOURNAL OF ALLERGY AND CLINICAL IMMUNOLOGY, vol. 131, no. 3, 4 February 2013 (2013-02-04), AMSTERDAM, NL, pages 856 - 865.e9, XP055477091, ISSN: 0091-6749, DOI: 10.1016/j.jaci.2012.11.028
- ADAIKALAVAN RAMASAMY ET AL: "Genome-Wide Association Studies of Asthma in Population-Based Cohorts Confirm Known and Suggested Loci and Identify an Additional Association near HLA", PLOS ONE, vol. 7, no. 9, 28 September 2012 (2012-09-28), pages e44008, XP055477089, DOI: 10.1371/journal.pone.0044008
- DEBORAH A MEYERS ET AL: "Asthma genetics and personalised medicine", THE LANCET. RESPIRATORY MEDICINE, vol. 2, no. 5, 1 May 2014 (2014-05-01), Oxford, pages 405 - 415, XP055477094, ISSN: 2213-2600, DOI: 10.1016/S2213-2600(14)70012-8

## Description

### FIELD

This disclosure relates generally to the field of precision medicine. More particularly, the disclosure relates to the detection of risk alleles in genes encoding IL33 and IL1RL1, which risk alleles can be used to stratify inflammatory lung disease patients as having a high risk of developing one or more of these conditions and their eosinophilic subtypes.

### BACKGROUND

Asthma and Chronic Obstructive Pulmonary Disease (COPD) are highly prevalent obstructive lung diseases with substantial unmet clinical need and significant diagnostic overlap, and there is increasing interest in the intersection of these conditions, termed asthma-COPD overlap syndrome (ACOS). There is a long-standing debate as to whether the two diseases have a shared etiology (the so-called "Dutch Hypothesis") or have independent mechanistic causes (the so-called "British Hypothesis). Despite recent progress in elucidating genetic contribution to common complex disease risk, including obstructive lung diseases, there is no well-established genetic finding linking asthma to COPD.

Genome-wide association studies (GWAS) have identified common genetic variants at interleukin-33 (IL33) and/or IL1RL1 that are associated with asthma. IL33, a pro-inflammatory cytokine and member of the interleukin-1 (IL-1) cytokine family, is expressed in subsets of cells in barrier tissues, including the lung epithelium. IL33 signals via a heterodimeric receptor complex composed of the IL33-specific receptor IL1RL1 (also known as ST2 or IL33R) and the IL-1RAcP co-receptor, common to several receptors of the IL-1 family.

In damaged tissues, previously sequestered IL33 is passively released into the extracellular compartment by necrotic cells and functions as an endogenous "danger signal" (alarmin) that activates inflammatory and repair pathways. Cigarette smoke induces IL33 expression in lung epithelial cells in mice, and IL33 expression is elevated in the bronchial epithelium of both asthma and COPD patients. In disease states in which inflammatory infiltrate and inflammatory cytokines are already present, the pool of IL33-responsive cells is increased and IL33 signaling further amplifies immune responses, resulting in pathologic inflammation and exaggerated immune responses, potentially driving chronic inflammatory diseases such as COPD. US 2016/168640 A1 (KHOSLA RAJITA [US] ET AL) is directed to methods of treating patients suffering from interleukin-33 (IL-33)-mediated disorders and methods of determining whether a patient is at increased risk for an IL-33-mediated disorder.

There is also an asthma-COPD overlap syndrome (ACOS), characterized by symptoms common to both asthma and COPD. Nevertheless, clinical challenges remain in the capacity to diagnose ACOS, given the difficulty in separating asthma from COPD owing to the overlapping features in common.

Treatment challenges for asthma, COPD, and ACOS also remain, with resistance to corticosteroids (the standard of care) fairly commonplace. As well, other treatments such as IL-5 therapy has not worked well for the eosinophilic subsets of asthma and COPD.

Accordingly, there remains a need in the art to distinguish among asthma, COPD, and ACOS, as well as to more accurately identify patients who have the eosinophilic subsets of these disorders. Proper diagnoses can better direct a therapeutic regimen and improve patient outcomes.

### SUMMARY

The invention is defined in the appended claims.

In a first aspect of the disclosure that is not encompassed by the wording of the claims, a method for treating or inhibiting eosinophilic asthma comprises administering an IL33 antagonist or administering an IL33 antagonist and an IL-4R antagonist to a subject having one or more risk alleles associated with eosinophilic asthma in the intronic IL1RL1 variant rs1420101 (SEQ ID NO: 357) or variant in linkage disequilibrium thereof, in the IL33 variant rs1342326 (SEQ ID NO: 358) or variant in linkage disequilibrium thereof, or in both the intronic IL1RL1 variant rs1420101 (SEQ ID NO: 357) or variant in linkage disequilibrium thereof and the IL33 variant rs1342326 (SEQ ID NO: 358) or variant in linkage disequilibrium thereof. Administration of an IL33 antagonist and/or an IL-4R antagonist is such that eosinophilic asthma is treated or inhibited in the subject.

In a second aspect of the disclosure that is not encompassed by the wording of the claims, a method for treating or inhibiting eosinophilic Chronic Obstructive Pulmonary Disease (COPD) comprises administering an IL33 antagonist or administering an IL33 antagonist and an IL-4R antagonist to a subject having one or more risk alleles associated with eosinophilic COPD in the intronic IL1RL1 variant rs1420101 (SEQ ID NO: 357) or variant in linkage disequilibrium thereof, in the IL33 variant rs1342326 (SEQ ID NO: 358) or variant in linkage disequilibrium thereof, or in both the intronic IL1RL1 variant rs1420101 (SEQ ID NO: 357) or variant in linkage disequilibrium thereof and the IL33 variant rs1342326 (SEQ ID NO: 358) or variant in linkage disequilibrium thereof. Administration of an IL33 antagonist and/or an IL-4R antagonist is such that eosinophilic COPD is treated or inhibited in the subject.

In a third aspect of the disclosure that is not encompassed by the wording of the claims, a method for treating or inhibiting eosinophilic asthma-Chronic Obstructive Pulmonary Disease (COPD) overlap syndrome (ACOS), comprising administering an IL33 antagonist or administering an IL33 antagonist and an IL-4R antagonist to a subject having one or more risk alleles associated with eosinophilic asthma in the intronic IL1RL1 variant rs1420101 (SEQ ID NO: 357) or variant in linkage disequilibrium thereof, in the IL33 variant rs1342326 (SEQ ID NO: 358) or variant in linkage disequilibrium thereof, or in both the intronic IL1RL1 variant rs1420101 (SEQ ID NO: 357) or variant in linkage disequilibrium thereof and the IL33 variant rs1342326 (SEQ ID NO: 358) or variant in linkage disequilibrium thereof. Administration of an IL33 antagonist and/or an IL-4R antagonist is such that eosinophilic COPD is treated or inhibited in the subject.

In a fourth aspect of the disclosure that is not encompassed by the wording of the claims, a method for treating or inhibiting nasal polyps comprises administering an IL33 antagonist or administering an IL33 antagonist and an IL-4R antagonist to a subject having one or more risk alleles associated with nasal polyps in the intronic IL1RL1 variant rs1420101 (SEQ ID NO: 357) or variant in linkage disequilibrium thereof, in the IL33 variant rs1342326 (SEQ ID NO: 358) or variant in linkage disequilibrium thereof, or in both the intronic IL1RL1 variant rs1420101 (SEQ ID NO: 357) or variant in linkage disequilibrium thereof and the IL33 variant rs1342326 (SEQ ID NO: 358) or variant in linkage disequilibrium thereof. Administration of an IL33 antagonist and/or an IL-4R antagonist is such that nasal polyps are treated or inhibited in the subject.

In a fifth aspect of the disclosure that is not encompassed by the wording of the claims, a method for assessing risk of development of eosinophilic asthma, eosinophilic Chronic Obstructive Pulmonary Disease (COPD), or eosinophilic asthma COPD overlap syndrome (ACOS) comprises the steps of:
(A) detecting one or more risk alleles associated with eosinophilic asthma, eosinophilic COPD, or eosinophilic ACOS in the intronic IL1RL1 variant rs1420101 (SEQ ID NO: 357) or variant in linkage disequilibrium thereof, in the IL33 variant rs1342326 (SEQ ID NO: 358) or variant in linkage disequilibrium thereof, or in both the intronic IL1RL1 variant rs1420101 (SEQ ID NO: 357) or variant in linkage disequilibrium thereof and the IL33 variant rs1342326 (SEQ ID NO: 358) or variant in linkage disequilibrium thereof in a sample obtained from a subject;
(B) (i) assigning a risk score of 1 to the subject when the subject has a risk allele in the intronic IL1RL1 variant rs1420101 (SEQ ID NO: 357) or variant in linkage disequilibrium thereof in one of the chromosome 2 homologs or a risk allele in the IL33 variant rs1342326 (SEQ ID NO: 358) or variant in linkage disequilibrium thereof in one of the chromosome 9 homologs,
(B) (ii) assigning a risk score of 2 to the subject when the subject has a risk allele in the intronic IL1RL1 variant rs1420101 (SEQ ID NO: 357) or variant in linkage disequilibrium thereof in both of the chromosome 2 homologs, when the subject has a risk allele in the IL33 variant rs1342326 (SEQ ID NO: 358) or variant in linkage disequilibrium thereof in both of the chromosome 9 homologs, or when the subject has a risk allele in the intronic IL1RL1 variant rs1420101 (SEQ ID NO: 357) or variant in linkage disequilibrium thereof in one of the chromosome 2 homologs and a risk allele in the IL33 variant rs1342326 (SEQ ID NO: 358) or variant in linkage disequilibrium thereof in one of the chromosome 9 homologs,
(B) (iii) assigning a risk score of 3 to the subject when the subject has a risk allele in the intronic IL1RL1 variant rs1420101 (SEQ ID NO: 357) or variant in linkage disequilibrium thereof in both of the chromosome 2 homologs and a risk allele in the IL33 variant rs1342326 (SEQ ID NO: 358) or variant in linkage disequilibrium thereof in one of the chromosome 9 homologs, or when the subject has a risk allele in the intronic IL1RL1 variant rs1420101 (SEQ ID NO: 357) or variant in linkage disequilibrium thereof in one of the chromosome 2 homologs and a risk allele in the IL33 variant rs1342326 (SEQ ID NO: 358) or variant in linkage disequilibrium thereof in both of the chromosome 9 homologs, or
(B) (iv) assigning a risk score of 4 to the subject when the subject has a risk allele in the intronic IL1RL1 variant rs1420101 (SEQ ID NO: 357) or variant in linkage disequilibrium thereof in both of the chromosome 2 homologs and a risk allele in the IL33 variant rs1342326 (SEQ ID NO: 358) or variant in linkage disequilibrium thereof in both of the chromosome 9 homologs; and
(C) categorizing the subject's risk of development of eosinophilic asthma, eosinophilic COPD, or eosinophilic ACOS, wherein a risk score of 1 indicates that the subject has a risk of developing the high-eosinophilic subset of eosinophilic asthma, high-eosinophilic subset of eosinophilic COPD, or high-eosinophilic subset of eosinophilic ACOS, a risk score of 2 indicates that the subject has an elevated risk of developing the high-eosinophilic subset of eosinophilic asthma, high-eosinophilic subset of eosinophilic COPD, or high-eosinophilic subset of eosinophilic ACOS, a risk score of 3 indicates that the subject has a high risk of developing the high-eosinophilic subset of eosinophilic asthma, high-eosinophilic subset of eosinophilic COPD, or high-eosinophilic subset of eosinophilic ACOS, and a risk score of 4 indicates that the subject has a very high risk of developing the high-eosinophilic subset of eosinophilic asthma, high-eosinophilic subset of eosinophilic COPD, or high-eosinophilic subset of eosinophilic ACOS. The method may further comprise treating or inhibiting one or more of the eosinophilic asthma, eosinophilic COPD, or eosinophilic ACOS, including the high eosinophilic subset thereof, in the subject by administering to the subject an IL33 antagonist or an IL33 antagonist and an IL-4R antagonist.

In a sixth aspect of the disclosure that is not encompassed by the wording of the claims, an IL33 antagonist or a combination of an IL33 antagonist and an IL-4R antagonist is for use in the treatment or inhibition of, or in the manufacture of a medicament for the treatment or inhibition of any one of eosinophilic asthma, eosinophilic Chronic Obstructive Pulmonary Disease (COPD), high-eosinophil eosinophilic asthma, high-eosinophil eosinophilic COPD, or nasal polyps when a patient thereof has one or more risk alleles associated with eosinophilic asthma, eosinophilic COPD, or eosinophilic ACOS in the intronic IL1RL1 variant rs1420101 (SEQ ID NO: 357) or variant in linkage disequilibrium thereof, in the IL33 variant rs1342326 (SEQ ID NO: 358) or variant in linkage disequilibrium thereof, or in both the intronic IL1RL1 variant rs1420101 (SEQ ID NO: 357) or variant in linkage disequilibrium thereof and the IL33 variant rs1342326 (SEQ ID NO: 358) or variant in linkage disequilibrium thereof.

According to any one of these aspects that are not encompassed by the wording of the claims, the subject may have at least one risk allele associated with eosinophilic asthma in the intronic IL1RL1 variant rs1420101 (SEQ ID NO: 357) or variant in linkage disequilibrium thereof, may have two risk alleles associated with eosinophilic asthma in the intronic IL1RL1 variant rs1420101 (SEQ ID NO: 357) or variant in linkage disequilibrium thereof, may have at least one risk allele associated with eosinophilic asthma in the IL33 variant rs1342326 (SEQ ID NO: 358) or variant in linkage disequilibrium thereof, or may have two risk alleles associated with eosinophilic asthma in the IL33 variant rs1342326 (SEQ ID NO: 358) or variant in linkage disequilibrium thereof, and may have further at least one risk allele associated with eosinophilic asthma in the IL33 variant rs1342326 (SEQ ID NO: 358) or variant in linkage disequilibrium thereof, and/or may further have two risk alleles associated with eosinophilic asthma in the IL33 variant rs1342326 (SEQ ID NO: 358) or variant in linkage disequilibrium thereof.

According to any one of these aspects that are not encompassed by the wording of the claims, the method may comprise or the use may be for administering an IL33 antagonist to the subject or the method may comprise or the use may be for administering an IL33 antagonist and an IL-4R antagonist to the subject. The IL33 antagonist may comprise an IL33 trap or an antibody that specifically binds to IL33. The IL-4R antagonist may comprise an antibody that specifically binds to IL-4R.

According to any of these aspects that are not encompassed by the wording of the claims, the IL33 trap may comprise a first IL33 binding domain comprising an IL33 binding portion of IL1RL1 and a second IL33 binding domain comprising an extracellular portion of IL-1RAcP. According to any of these aspects, the antibody or antigen-binding fragment thereof that specifically binds to IL33 may comprise the H1, H2, and H3 domains of SEQ ID NO: 274 and the L1, L2, and L3 domains of SEQ ID NO: 282. According to any of these aspects, the antibody or antigen-binding fragment thereof that specifically binds to IL-4R may comprise the H1, H2, and H3 domains of SEQ ID NO: 337 and the L1, L2, and L3 domains of SEQ ID NO: 338.

### BRIEF DESCRIPTION OF THE DRAWINGS

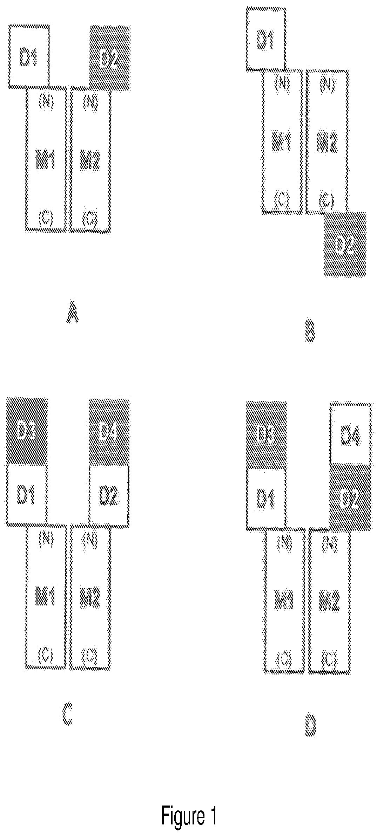

Figure 1 (Panels A, B, C, and D) shows four exemplary arrangements of the individual components of the IL33 antagonists relative to one another. Panel A shows an arrangement in which a first IL33-binding domain (D1) is attached to the N-terminus of a first multimerizing domain (M1), and a second IL33-binding domain (D2) is attached to the N-terminus of a second multimerizing domain (M2). D1 is shown as a white box and D2 is shown as a black box to indicate that D1 and D2 are derived from different IL33 binding proteins. Panel B shows an arrangement in which a first IL33-binding domain (D1) is attached to the N-terminus of a first multimerizing domain (M1), and a second IL33-binding domain (D2) is attached to the C-terminus of a second multimerizing domain (M2). D1 is shown as a white box and D2 is shown as a black box to indicate that D1 and D2 are derived from different IL33 binding proteins. Panels C and D show arrangements comprising four IL33-binding domains, D1, D2, D3 and D4. In these arrangements, D3-D1-M1 and D4-D2-M2 are attached in tandem, wherein D3 is attached to the N-terminus of D1, and D1 is attached to the N-terminus of M1; and D4 is attached to the N-terminus of D2, and D2 is attached to the N-terminus of M2. In Panel C, D3 and D4 are identical or substantially identical to one another, and D1 and D2 are identical or substantially identical to one another. In Panel D, D1 and D4 are identical or substantially identical to one another, and D3 and D2 are identical or substantially identical to one another.

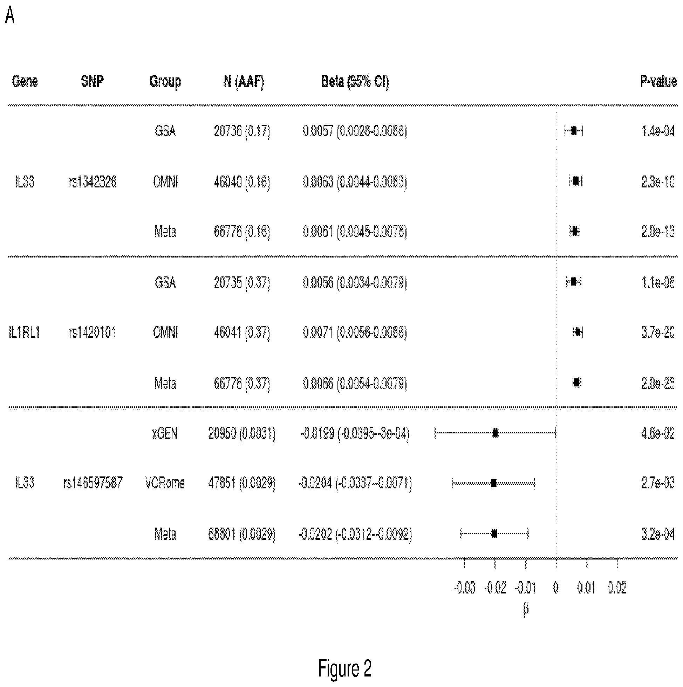

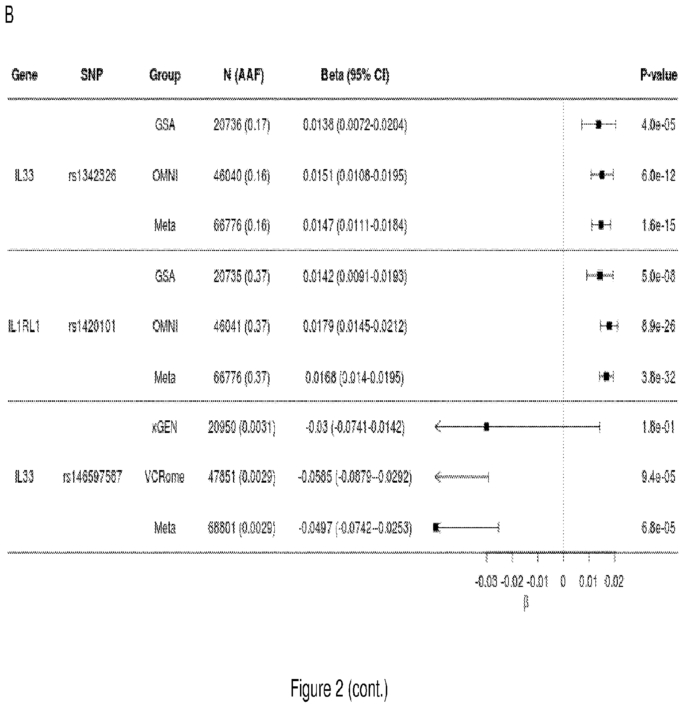

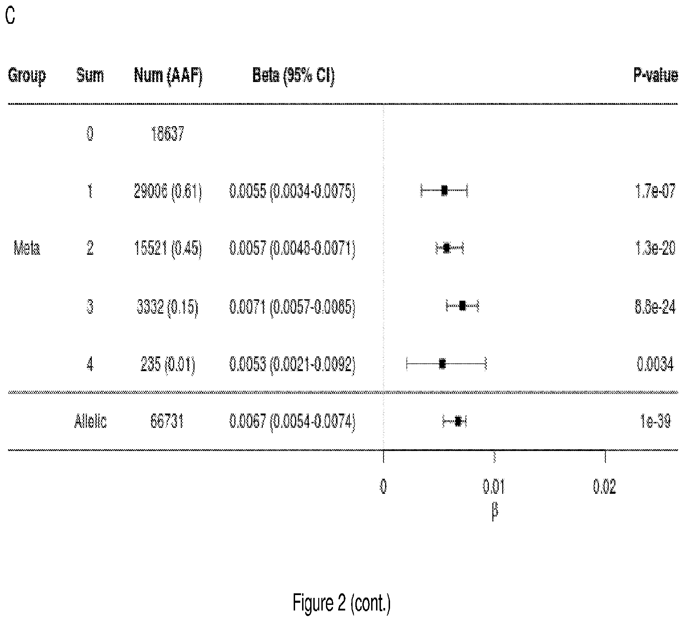

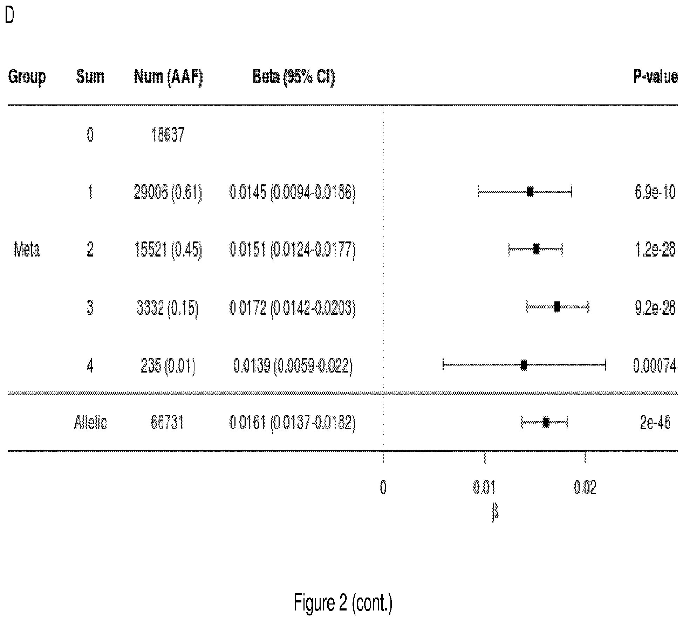

Figure 2 (Panels A, B, C, and D) shows rs1420101 (*IL1RL1*, also known as ST2), s1342326 (*IL33*) and rs146597587 (*IL33-*pLoF) associations with (Panel A) eosinophil counts (Panel B) log₁₀ eosinophil counts. The associations between the total burden of rs1420101 and rs1342326 risk alleles, and (Panel C) eosinophil counts (Panel D) log₁₀ eosinophil counts is also shown. Effect sizes and P-values for eosinophil counts and log₁₀ eosinophil counts, were calculated using linear regression, with adjustment for age, age², sex, smoking status and principal components of ancestry. P-values and effect sizes/odds ratios were estimated for individual scores; in each case the comparison was to individuals with zero risk alleles. Additionally, overall allelic effects p-values are shown.

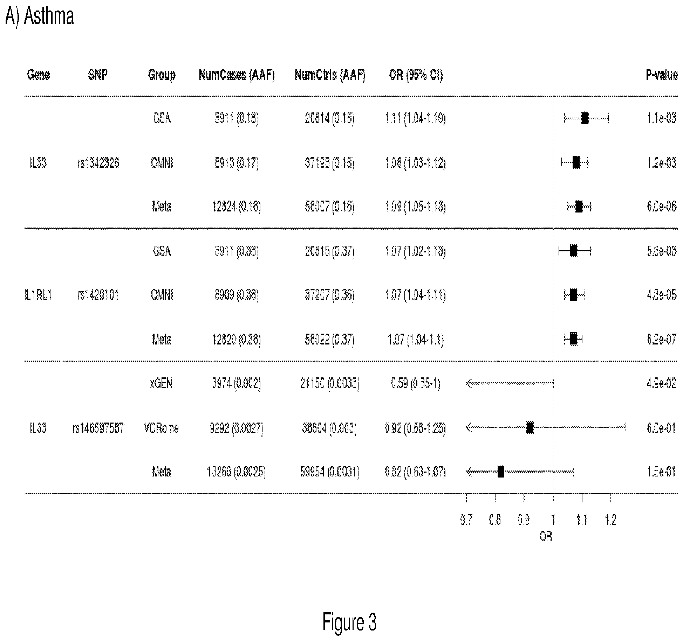

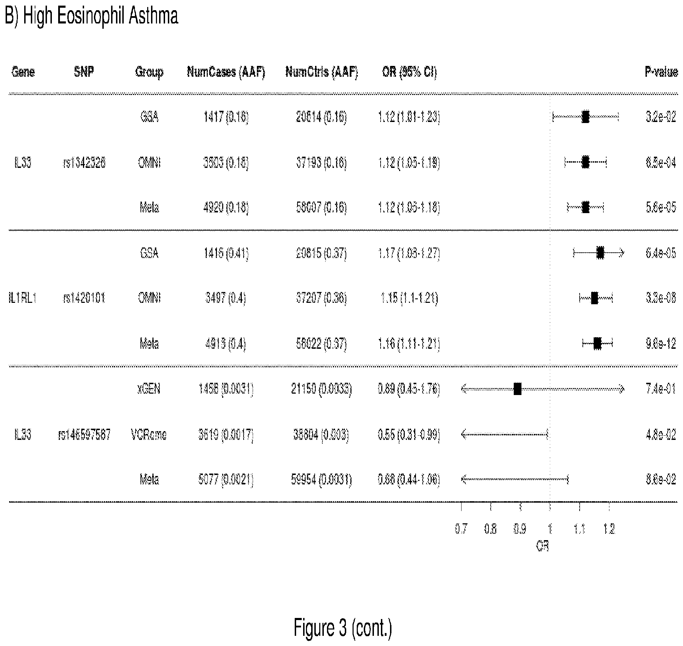

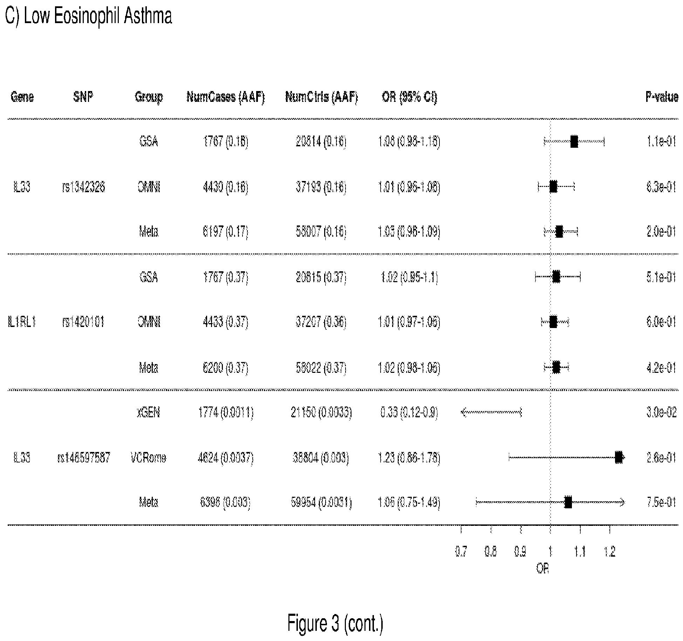

Figure 3 (Panels A, B, and C) shows rs1420101 (*IL1RL1*, also known as ST2), s1342326 (*IL33*) and rs146597587 (*IL33-*pLoF) associations with (Panel A) Asthma, (Panel B) High Eosinophil Asthma Subset and (Panel C) Low Eosinophil Asthma Subset. Odds ratios for disease were calculated using logistic regression, with adjustment for age, age², sex, smoking status and principal components of ancestry.

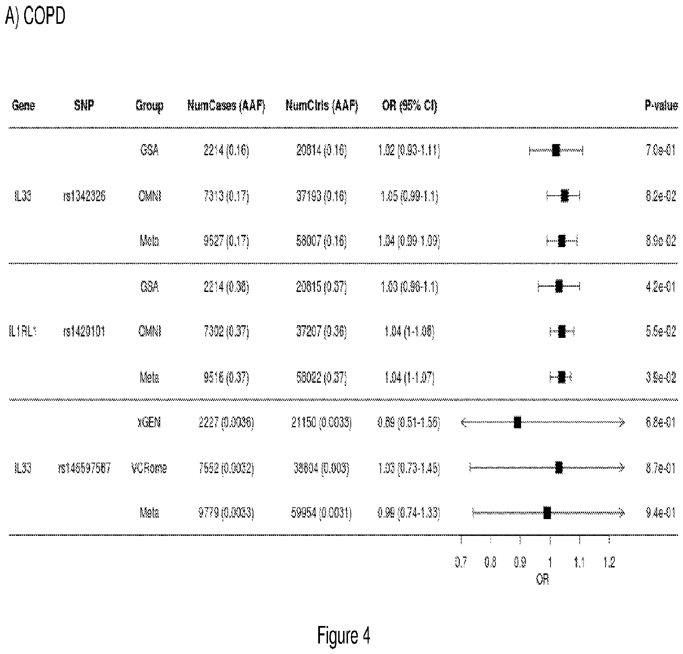

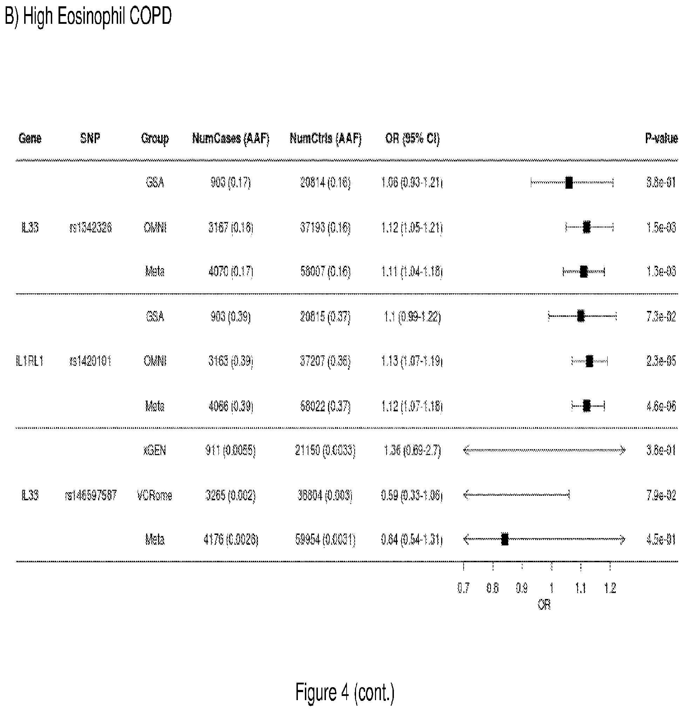

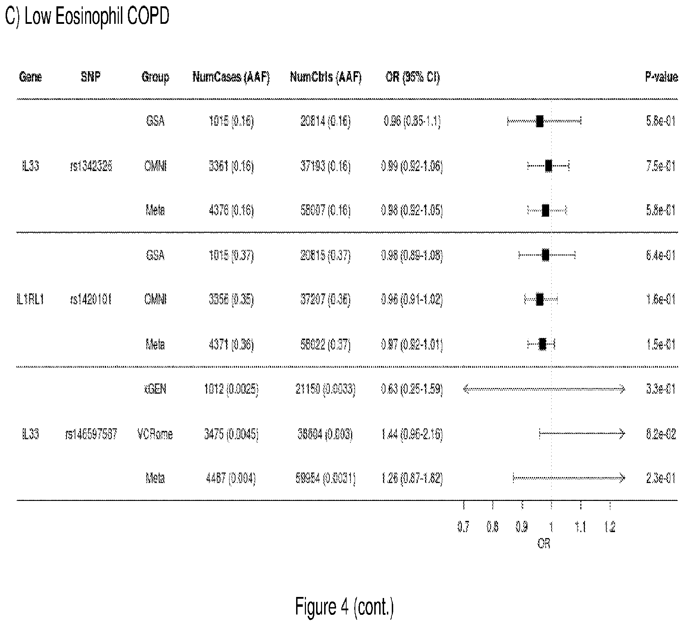

Figure 4 (Panels A, B, and C) shows rs1420101 (*IL1RL1*, also known as ST2), s1342326 (*IL33*) and rs146597587 (*IL33-*pLoF) associations with (Panel A) COPD, (Panel B) High Eosinophil COPD Subset and (Panel C) Low Eosinophil COPD Subset. Odds ratios for disease were calculated using logistic regression, with adjustment for age, age², sex, smoking status and principal components of ancestry.

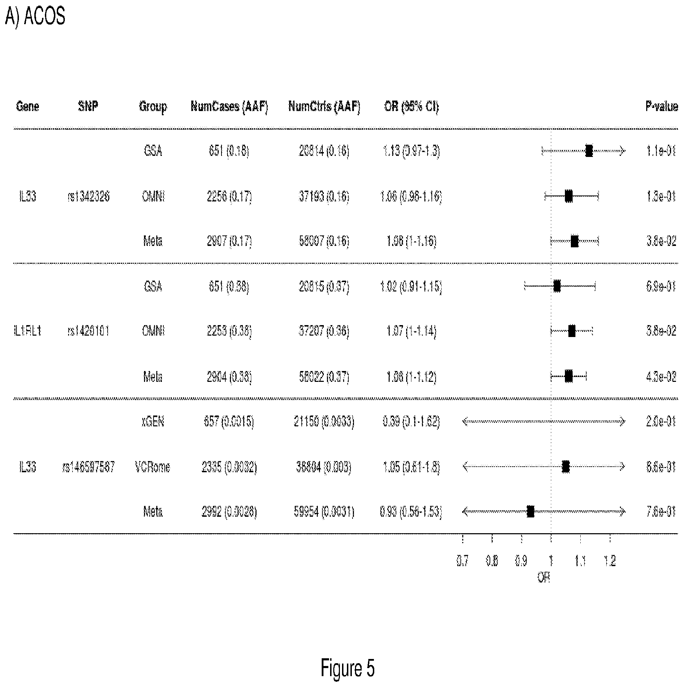

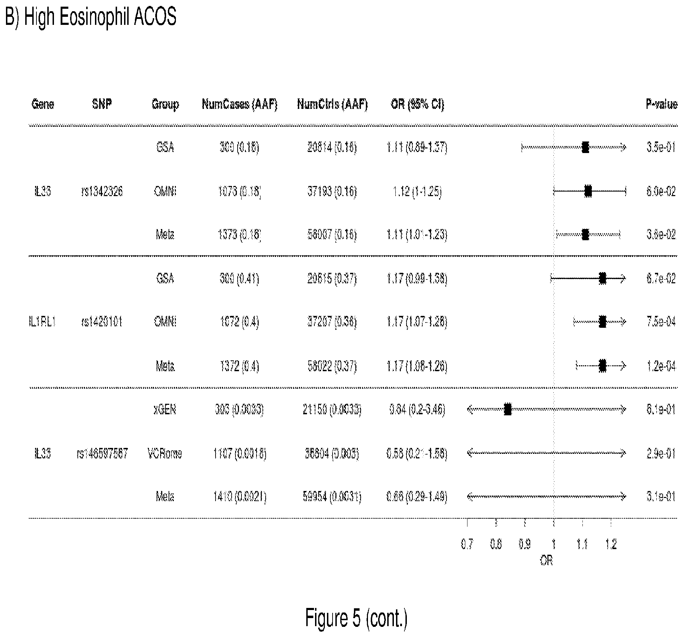

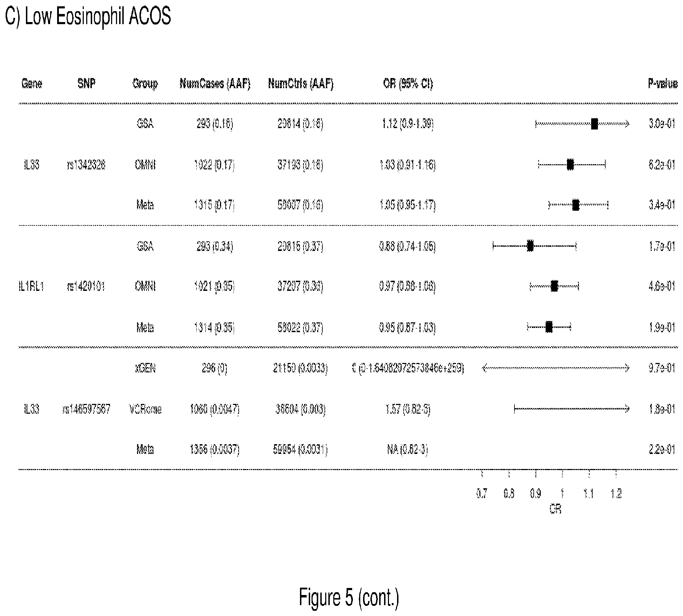

Figure 5 (Panels A, B, and C) shows rs1420101 (*IL1RL1*, also known as ST2), s1342326 (*IL33*) and rs146597587 (*IL33-*pLoF) associations with (Panel A) ACOS, (Panel B) High Eosinophil ACOS Subset and (Panel C) Low Eosinophil ACOS Subset. Odds ratios for disease were calculated using logistic regression, with adjustment for age, age², sex, smoking status and principal components of ancestry.

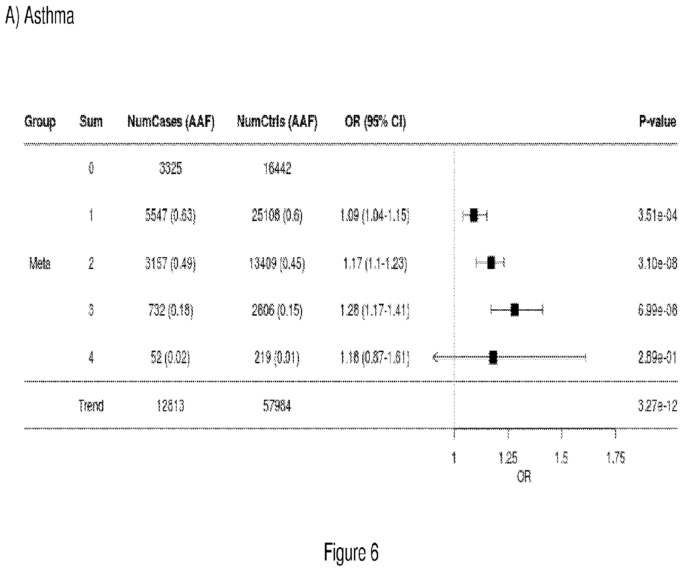

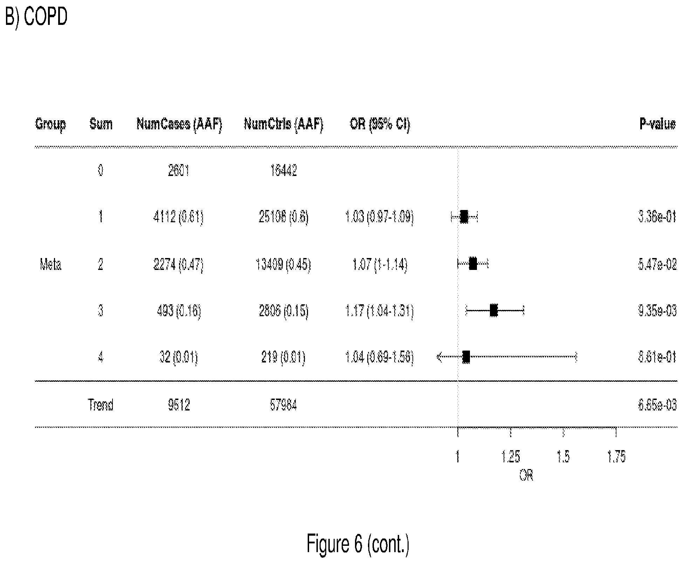

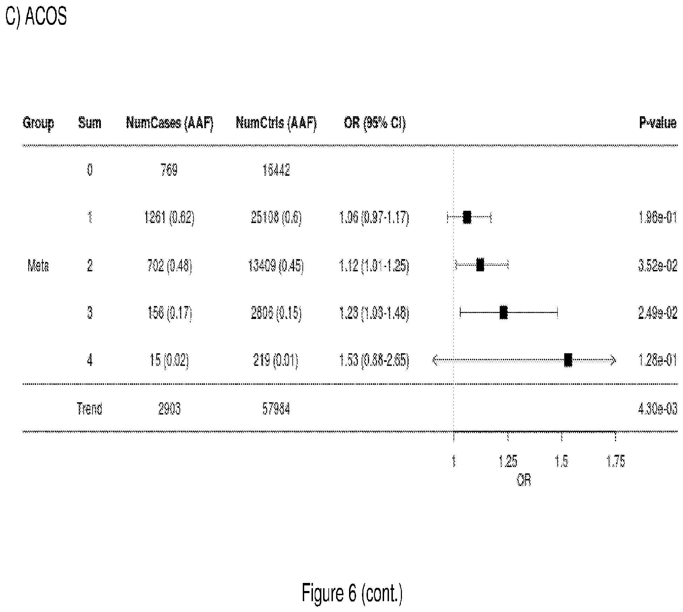

Figure 6 (Panels A, B, and C) shows genetic score (total burden of rs1420101 and rs1342326 risk alleles) associations with (Panel A) Asthma (Panel B) COPD and (Panel C) ACOS. P-values odds ratios were estimated for individual scores; in each case the comparison was to individuals with zero risk alleles. Additionally, overall trend test p-values are shown.

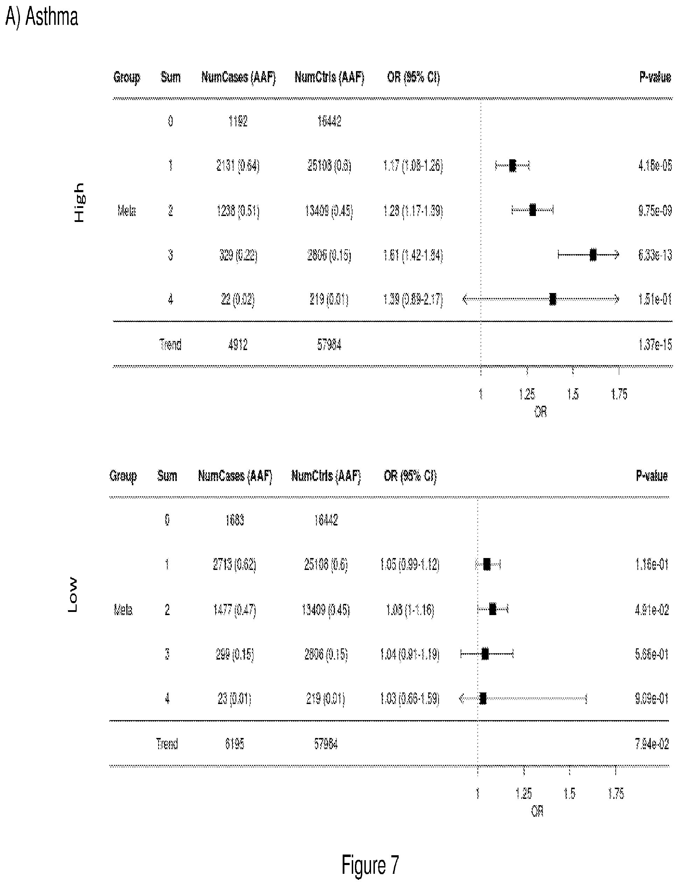

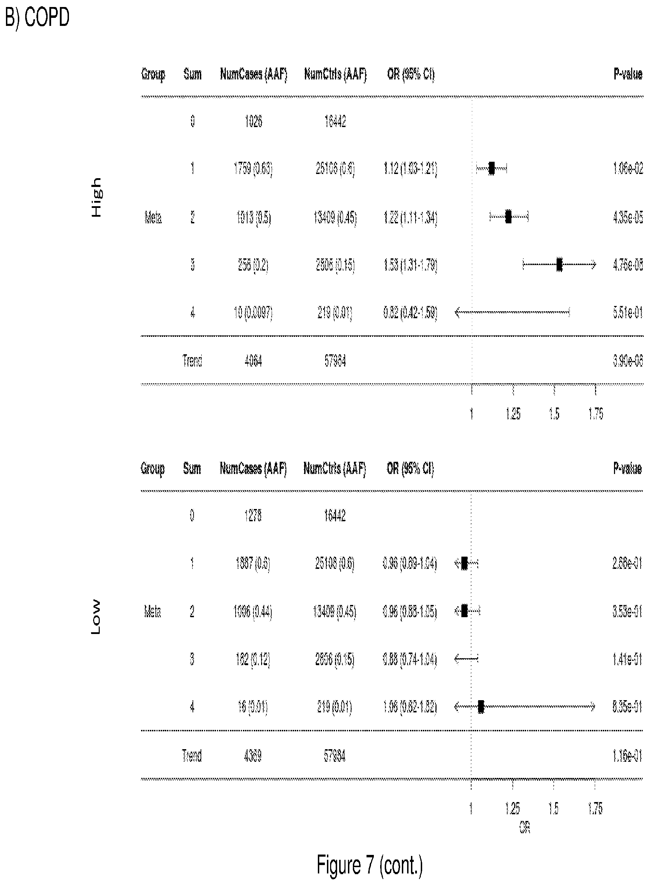

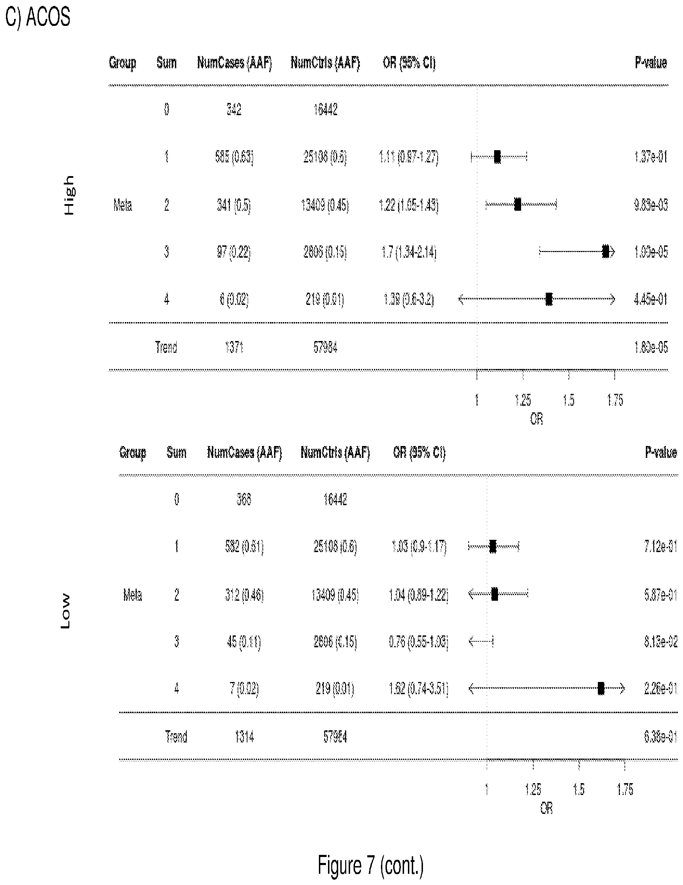

Figure 7 (Panels A, B, and C) shows genetic score (total burden of rs1420101 and rs1342326 risk alleles) associations with (Panel A) High and Low Eosinophil Asthma (Panel B) High and Low Eosinophil COPD and (Panel C) High and Low Eosinophil ACOS. P-values odds ratios were estimated for individual scores; in each case the comparison was to individuals with zero risk alleles. Additionally, overall trend test p-values are shown.

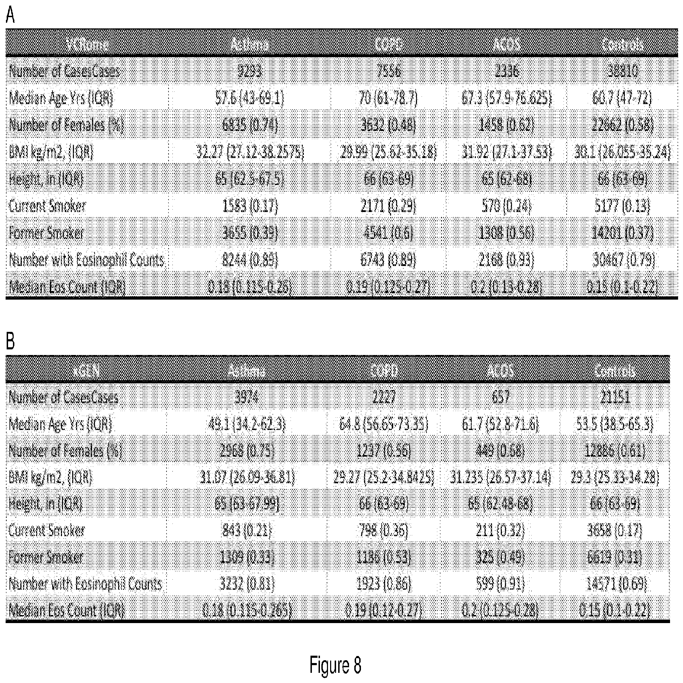

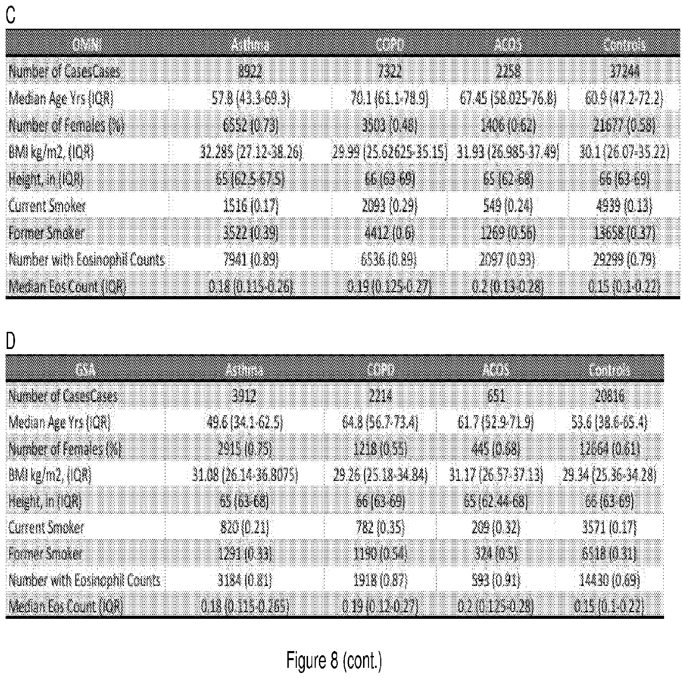

Figure 8 (Panels A, B, C, and D) shows the clinical characteristics of study participants stratified by capture reagent (Panel A) VCRome and (Panel B) xGEN, and chip platform (Panel C) OMNI and (Panel D) GSA.

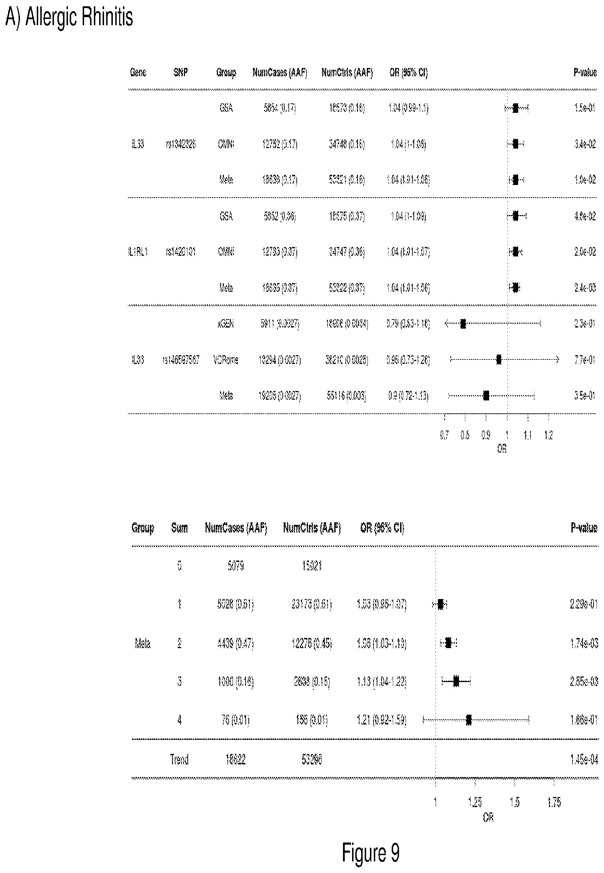

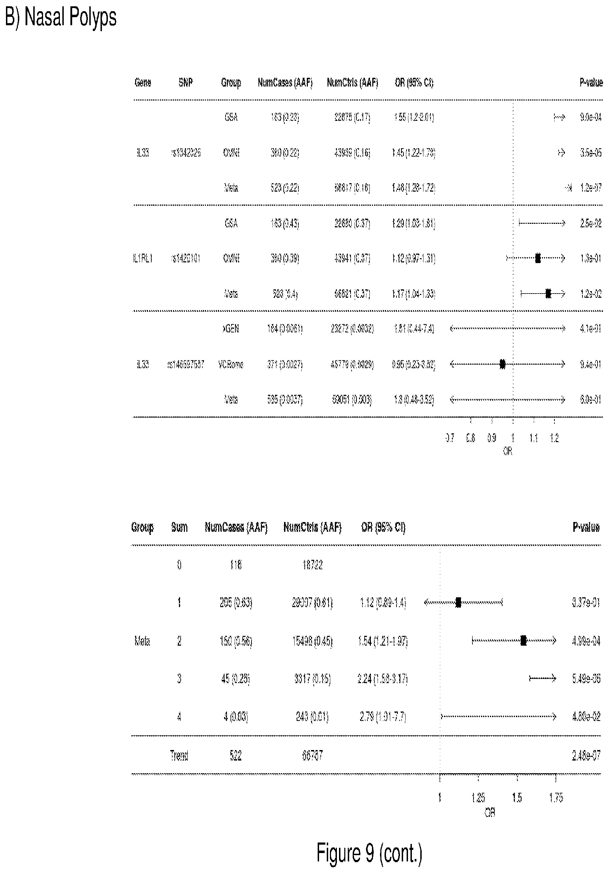

Figure 9 (Panels A and B) shows rs1420101 (*IL1RL1*, also known as ST2), s1342326 (*IL33*), rs146597587 (*IL33-*pLoF) and genetic score (total burden of rs1420101 and rs1342326 risk alleles) associations with (Panel A) Allergic Rhinitis and (Panel B) Nasal Polyps. Odds ratios for disease were calculated using logistic regression, with adjustment for age, age², sex, smoking status and principal components of ancestry. To test the burden of common risk variants, p-values and odds ratios were estimated for each individual score; in each case the comparison was to individuals with zero risk alleles. Additionally, overall trend test p-values are shown.

### DETAILED DESCRIPTION

Various terms relating to aspects of disclosure are used throughout the specification and claims. Such terms are to be given their ordinary meaning in the art, unless otherwise indicated. Other specifically defined terms are to be construed in a manner consistent with the definition provided herein.

As used herein, the singular forms "a," "an," and "the" include plural referents unless expressly stated otherwise.

The terms "subject" and "patient" are used interchangeably and include any animal. Mammals are preferred, including companion *(e.g.,* cat, dog) and farm mammals *(e.g.,* pig, horse, cow), as well as rodents, including mice, rabbits, and rats, guinea pigs, and other rodents. Non-human primates are more preferred, and human beings are highly preferred.

The term "isolated" means removed and/or altered from the natural environment by the hand of a human being.

A "risk allele" includes alternative polymorphisms at a particular position that associate with a risk of developing a disease, disorder, or condition.

"Linkage disequilibrium" refers to a nonrandom association of alleles at two or more loci.

It has been observed in accordance with the disclosure that single nucleotide polymorphisms in *IL1R1* rs1420101 (SEQ ID NO: 357) and *IL33* rs1342326 (SEQ ID NO: 358) associate with increased risk of asthma, as well as high-eosinophil subsets of asthma, COPD, and ACOS. In addition, it was observed that individuals carrying a larger burden of these risk alleles across both loci have an attendant larger disease risk and, heterozygous carriers of rare pLOF variants in *IL33* had lower median lifetime eosinophil counts and trends reflecting decreased risk of asthma, as well as trends reflecting decreased risks high-eosinophil subsets of asthma, COPD, and ACOS. It is believed that *IL33* pathway genetic variants have not been previously associated with COPD and, it is further believed that there have not been any reported genetic links between asthma and COPD or genetic links between the *IL33* pathway the risk of high-eosinophil subsets of asthma, COPD and ACOS. Furthermore, it was observed that single nucleotide polymorphisms in *IL1R1* rs1420101 and *IL33* rs1342326, considered individually and in aggregate, associate with increased risk of nasal polyps and allergic rhinitis. These data indicate a role for interleukin-33 blockade in the treatment of high-eosinophil forms of obstructive lung diseases such as asthma, COPD, and ACOS, as well as other upper airways diseases such as nasal polyps and their high-eosinophil subsets. Accordingly, the disclosure features methods for identifying risk, diagnosing, treating, and inhibiting asthma, COPD, and ACOS, especially the high-eosinophil subsets thereof.

In a first aspect, the disclosure features methods for assessing risk of development of an inflammatory lung disease. The inflammatory lung disease may be one or more of asthma, COPD, ACOS, or nasal polyps. The asthma may be eosinophilic asthma or high-eosinophil eosinophilic asthma. The COPD may be eosinophilic COPD or high-eosinophil eosinophilic COPD. The ACOS may be eosinophilic ACOS or high-eosinophil eosinophilic ACOS. In general, the methods comprise detecting one or more risk alleles associated with risk of development of such inflammatory lung diseases.

In some embodiments, the methods comprise detecting one or more risk alleles associated with eosinophilic asthma, eosinophilic COPD, eosinophilic ACOS, or nasal polyps in the intronic IL1RL1 variant rs1420101 (SEQ ID NO: 357) or variant in linkage disequilibrium thereof, in the IL33 variant rs1342326 (SEQ ID NO: 358) or variant in linkage disequilibrium thereof, or in both the intronic IL1RL1 variant rs1420101 (SEQ ID NO: 357) or variant in linkage disequilibrium thereof and the IL33 variant rs1342326 (SEQ ID NO: 358) or variant in linkage disequilibrium thereof in a sample obtained from a subject, then assigning a risk score of 1 to the subject when the subject has a risk allele in the intronic IL1RL1 variant rs1420101 (SEQ ID NO: 357) or variant in linkage disequilibrium thereof in one of the chromosome 2 homologs or a risk allele in the IL33 variant rs1342326 (SEQ ID NO: 358) or variant in linkage disequilibrium thereof in one of the chromosome 9 homologs, assigning a risk score of 2 to the subject when the subject has a risk allele in the intronic IL1RL1 variant rs1420101 (SEQ ID NO: 357) or variant in linkage disequilibrium thereof in both of the chromosome 2 homologs, when the subject has a risk allele in the IL33 variant rs1342326 (SEQ ID NO: 358) or variant in linkage disequilibrium thereof in both of the chromosome 9 homologs, or when the subject has a risk allele in the intronic IL1RL1 variant rs1420101 (SEQ ID NO: 357) or variant in linkage disequilibrium thereof in one of the chromosome 2 homologs and a risk allele in the IL33 variant rs1342326 (SEQ ID NO: 358) or variant in linkage disequilibrium thereof in one of the chromosome 9 homologs, assigning a risk score of 3 to the subject when the subject has a risk allele in the intronic IL1RL1 variant rs1420101 (SEQ ID NO: 357) or variant in linkage disequilibrium thereof in both of the chromosome 2 homologs and a risk allele in the IL33 variant rs1342326 (SEQ ID NO: 358) or variant in linkage disequilibrium thereof in one of the chromosome 9 homologs, or when the subject has a risk allele in the intronic IL1RL1 variant rs1420101 (SEQ ID NO: 357) or variant in linkage disequilibrium thereof in one of the chromosome 2 homologs and a risk allele in the IL33 variant rs1342326 (SEQ ID NO: 358) or variant in linkage disequilibrium thereof in both of the chromosome 9 homologs, or assigning a risk score of 4 to the subject when the subject has a risk allele in the intronic IL1RL1 variant rs1420101 (SEQ ID NO: 357) or variant in linkage disequilibrium thereof in both of the chromosome 2 homologs and a risk allele in the IL33 variant rs1342326 (SEQ ID NO: 358) or variant in linkage disequilibrium thereof in both of the chromosome 9 homologs.

In some embodiments, the risk allele in the *IL33* variant rs1342326 comprises a single nucleotide polymorphism (SNP). In some detailed embodiments, the *IL33* variant comprises the SNP 9:6190076:A:C. (Human Genome GRCh38) The variant rs1342326 comprises the following nucleic acid sequence: CCAATCTTTTCTCATGAAGACACCA[G/T]CATGACCTCTTATTCTTA TTTATAT (SEQ ID NO: 358).

In some embodiments, the risk allele in the *IL1RL1* variant rs1420101 comprises an SNP. In some detailed embodiments, the IL1RL1 variant comprises the SNP 2:102341256:C:T (Human Genome GRCh38) . The variant rs1342326 comprises the following nucleic acid sequence: TATACCATCACAAAGCCTCTCATTA[A/G]ACTTTGAATCCAATGAGTATTACTA (SEQ ID NO: 357).

Detection may be according to any suitable methodology. The risk alleles may be detected, for example, by way of sequencing, genotyping, imputation, probing with complementary nucleic acid probes.

The methods may further comprise categorizing the subject's risk of development of eosinophilic asthma, eosinophilic COPD, or eosinophilic ACOS, wherein a risk score of 1 indicates that the subject has a risk of developing the high-eosinophil subset of eosinophilic asthma, high-eosinophil subset of eosinophilic COPD, or high-eosinophil subset of eosinophilic ACOS, a risk score of 2 indicates that the subject has an elevated risk of developing the high-eosinophil subset of eosinophilic asthma, high-eosinophil subset of eosinophilic COPD, or high-eosinophil subset of eosinophilic ACOS, a risk score of 3 indicates that the subject has a high risk of developing the high-eosinophil subset of eosinophilic asthma, high-eosinophil subset of eosinophilic COPD, or high-eosinophil subset of eosinophilic ACOS, and a risk score of 4 indicates that the subject has a very high risk of developing the high-eosinophil subset of eosinophilic asthma, high-eosinophil subset of eosinophilic COPD, or high-eosinophil subset of eosinophilic ACOS. In this scale, an elevated risk is greater than a risk but lesser than a high risk, and a very high risk is greater than a high risk. Thus, in terms of patient risk of developing disease, risk < elevated risk < high risk < very high risk, or risk score of 1 < risk score of 2 < risk score of 3 < risk score of 4.

The methods may further comprise obtaining a sample from the subject. In general, the sample may comprise any sample from which the risk alleles may be detected. The sample may comprise a tissue sample or sputum. A tissue sample may include peripheral blood, airway or lung tissue.

The methods may further comprise identifying the subject as a candidate for treatment with an IL33 antagonist or a combination of an IL33 antagonist and an IL-4R antagonist. Based on the categorization of the subject's risk as a risk score of 1, risk score of 2, risk score of 3, or risk score of 4, the subject may benefit from a therapeutic regimen that inhibits eosinophilic asthma, eosinophilic COPD, eosinophilic ACOS, or the high eosinophil subsets thereof, or nasal polyps. An inhibitory therapeutic regimen may comprise adjustments in type, dose, dosing frequency, etc. for the IL33 antagonist, as well as whether or not to combine with an IL-4R antagonist and, if so, the type, dose, and dosing frequency, etc. for the IL-4R antagonist, for example, depending on the level of risk.

The methods may further comprise detecting increased levels in eosinophil counts from blood or sputum isolated from the subject. Increased levels are those that are considered above normal levels or above levels typically observed in subjects that have the non-eosinophilic subset of asthma, COPD, or ACOS. The methods may further comprise isolating the blood or sputum from the subject for this purpose.

Without forming part of the invention, the methods may further comprise administering an IL33 antagonist or administering an IL33 antagonist and an IL-4R antagonist to the subject. Such administration may be according to an amount effective to inhibit eosinophilic asthma, eosinophilic COPD, or eosinophilic ACOS, or the high-eosinophil subset thereof. In some embodiments, the IL33 antagonist may comprise an IL33 trap. In some embodiments, the IL33 antagonist may comprise an antibody that specifically binds to IL33 or antigen-binding fragment thereof. Suitable IL33 antagonists are described herein. In some embodiments, the IL-4R antagonist may comprise an antibody that specifically binds to IL-4R or antigen-binding fragment thereof. Suitable IL-4R antagonists are described herein.

Without forming part of the invention, the disclosure features methods for treating or inhibiting eosinophilic asthma in a subject in need thereof. The eosinophilic asthma may be the low-eosinophil subset of eosinophilic asthma or may be the high eosinophil subset of eosinophilic asthma.

Without forming part of the invention, the methods comprise administering an IL33 antagonist or administering an IL33 antagonist and an IL-4R antagonist to a subject having one or more risk alleles associated with eosinophilic asthma in the intronic IL1RL1 variant rs1420101 (SEQ ID NO: 357) or variant in linkage disequilibrium thereof, in the IL33 variant rs1342326 (SEQ ID NO: 358) or variant in linkage disequilibrium thereof, or in both the intronic IL1RL1 variant rs1420101 (SEQ ID NO: 357) or variant in linkage disequilibrium thereof and the IL33 variant rs1342326 (SEQ ID NO: 358) or variant in linkage disequilibrium thereof, such that eosinophilic asthma is treated or inhibited in the subject.

In some embodiments, the IL33 antagonist may comprise an IL33 trap. In some embodiments, the IL33 antagonist may comprise an antibody that specifically binds to IL33 or antigen-binding fragment thereof. Suitable IL33 antagonists are described herein. In some embodiments, the IL-4R antagonist may comprise an antibody that specifically binds to IL-4R or antigen-binding fragment thereof. Suitable IL-4R antagonists are described herein.

Without forming part of the invention, the disclosure features methods for treating or inhibiting eosinophilic COPD in a subject in need thereof. The eosinophilic COPD may be the low-eosinophil subset of eosinophilic COPD or may be the high eosinophil subset of eosinophilic COPD.

Without forming part of the invention, the methods comprise administering an IL33 antagonist or administering an IL33 antagonist and an IL-4R antagonist to a subject having one or more risk alleles associated with eosinophilic COPD in the intronic IL1RL1 variant rs1420101 (SEQ ID NO: 357) or variant in linkage disequilibrium thereof, in the IL33 variant rs1342326 (SEQ ID NO: 358) or variant in linkage disequilibrium thereof, or in both the intronic IL1RL1 variant rs1420101 (SEQ ID NO: 357) or variant in linkage disequilibrium thereof and the IL33 variant rs1342326 (SEQ ID NO: 358) or variant in linkage disequilibrium thereof, such that eosinophilic COPD is treated or inhibited in the subject.

In some embodiments, the IL33 antagonist may comprise an IL33 trap. In some embodiments, the IL33 antagonist may comprise an antibody that specifically binds to IL33 or antigen-binding fragment thereof. Suitable IL33 antagonists are described herein. In some embodiments, the IL-4R antagonist may comprise an antibody that specifically binds to IL-4R or antigen-binding fragment thereof. Suitable IL-4R antagonists are described herein.

Without forming part of the invention, the disclosure features methods for treating or inhibiting eosinophilic ACOS in a subject in need thereof. The eosinophilic ACOS may be the low-eosinophil subset of eosinophilic ACOS or may be the high eosinophil subset of eosinophilic ACOS.

Without forming part of the invention, the methods comprise administering an IL33 antagonist or administering an IL33 antagonist and an IL-4R antagonist to a subject having one or more risk alleles associated with eosinophilic ACOS in the intronic IL1RL1 variant rs1420101 (SEQ ID NO: 357) or variant in linkage disequilibrium thereof, in the IL33 variant rs1342326 (SEQ ID NO: 358) or variant in linkage disequilibrium thereof, or in both the intronic IL1RL1 variant rs1420101 (SEQ ID NO: 357) or variant in linkage disequilibrium thereof and the IL33 variant rs1342326 (SEQ ID NO: 358) or variant in linkage disequilibrium thereof, such that eosinophilic ACOS is treated or inhibited in the subject.

In some embodiments, the IL33 antagonist may comprise an IL33 trap. In some embodiments, the IL33 antagonist may comprise an antibody that specifically binds to IL33 or antigen-binding fragment thereof. Suitable IL33 antagonists are described herein. In some embodiments, the IL-4R antagonist may comprise an antibody that specifically binds to IL-4R or antigen-binding fragment thereof. Suitable IL-4R antagonists are described herein.

Without forming part of the invention, an IL33 antagonist may be administered as part of a therapeutic regimen. An IL33 antagonist may comprise any agent that inhibits the interaction of IL33 with one or more of its binding partners and, in so doing, inhibit IL33-mediated signaling. For example, an IL33 antagonist may bind to and/or interact with IL33, or with the IL33 receptor referred to as "suppression of tumorigenicity" (aka ST2), or with the IL33 co-receptor Interleukin-1 Receptor Accessory Protein (IL-1RAcP), or with a complex of any of the following: IL33/ST2, or ST2/IL-1RAcP.

Non-limiting examples of categories of IL33 antagonists include small molecule IL33 inhibitors, or receptor antagonists, or nucleic acids that hybridize under stringent conditions to nucleic acid sequences encoding either IL33, or an IL33 receptor or co-receptor (e.g., short interfering RNAs (siRNA) or clustered regularly interspaced short palindromic repeat RNAs (CRISPR-RNA or crRNA), including single guide RNAs (sgRNAs) having a crRNA and tracrRNA sequence. Other IL33 antagonists include proteins comprising a ligand-binding portion of an IL33 receptor *(e.g.,* ST2), IL33-binding scaffold molecules *(e.g.,* DARPins, HEAT repeat proteins, ARM repeat proteins, tetratricopeptide repeat proteins, fibronectin-based scaffold constructs, and other scaffolds based on naturally occurring repeat proteins, and anti-IL33 aptamers or portions thereof.

In preferred embodiments, an IL33 antagonist comprises an antibody that specifically binds to human IL33 (IL33 antibodies), or antigen-binding fragments thereof. The amino acid sequence identifiers for exemplary anti-IL33 antibodies for use in the methods described herein are shown in Table 1. Anti-IL33 antibodies may comprise any antibody described in U.S. Pat. No. 9,453,072.

**Table 1. IL33 Antibodies Amino Acid Sequence Identifiers**

| | **SEQ ID NOs:** | | | | | | | |
|---|---|---|---|---|---|---|---|---|
| **Antibody Designation** | **HCVR** | **HCDR1** | **HCDR2** | **HCDR3** | **LCVR** | **LCDR1** | **LCDR2** | **LCDR3** |
| H1M9559N | 2 | 4 | 6 | 8 | 10 | 12 | 14 | 16 |
| H1M9566N | 18 | 20 | 22 | 24 | 26 | 28 | 30 | 32 |
| H1M9568N | 34 | 36 | 38 | 40 | 42 | 44 | 46 | 48 |
| H4H9629P | 50 | 52 | 54 | 56 | 58 | 60 | 62 | 64 |
| H4H9633P | 66 | 68 | 70 | 72 | 74 | 76 | 78 | 80 |
| H4H9640P | 82 | 84 | 86 | 88 | 90 | 92 | 94 | 96 |
| H4H9659P | 98 | 100 | 102 | 104 | 106 | 108 | 110 | 112 |
| H4H9660P | 114 | 116 | 118 | 120 | 122 | 124 | 126 | 128 |
| H4H9662P | 130 | 132 | 134 | 136 | 138 | 140 | 142 | 144 |
| H4H9663P | 146 | 148 | 150 | 152 | 154 | 156 | 158 | 160 |
| H4H9664P | 162 | 164 | 166 | 168 | 170 | 172 | 174 | 176 |
| H4H9665P | 178 | 180 | 182 | 184 | 186 | 188 | 190 | 192 |
| H4H9666P | 194 | 196 | 198 | 200 | 202 | 204 | 206 | 208 |
| H4H9667P | 210 | 212 | 214 | 216 | 218 | 220 | 222 | 224 |
| H4H9670P | 226 | 228 | 230 | 232 | 234 | 236 | 238 | 240 |
| H4H9671P | 242 | 244 | 246 | 248 | 250 | 252 | 254 | 256 |
| H4H9672P | 258 | 260 | 262 | 264 | 266 | 268 | 270 | 272 |
| H4H9675P | 274 | 276 | 278 | 280 | 282 | 284 | 286 | 288 |
| H4H9676P | 290 | 292 | 294 | 296 | 298 | 300 | 302 | 304 |
| H1M9565N | 308 | 310 | 312 | 314 | 316 | 318 | 320 | 322 |

In some embodiments, the IL33 antagonist comprises an anti-IL33 antibody, or antigen-binding fragment thereof, comprising a heavy chain variable region (HCVR), light chain variable region (LCVR), and/or complementarity determining regions (CDRs) of the amino acid sequences of the anti-IL33 antibodies as set forth in U.S. Pat. No. 9,453,072 and in Table 1 herein. In some embodiments, the IL33 antagonist comprises the heavy chain complementarity determining regions (CDR; *e.g.,* H1, H2, H3) of the heavy chain variable region comprising the amino acid sequence of SEQ ID NO: 274 and the light chain CDRs (e.g., L1, L2, L3) of the light chain variable region comprising the amino acid sequence of SEQ ID NO: 282. In some embodiments, the H1 comprises the amino acid sequence of SEQ ID NO: 276, the H2 comprises the amino acid sequence of SEQ ID NO: 278, and the H3 comprises the amino acid sequence of SEQ ID NO: 280. In some embodiments, the L1 comprises the amino acid sequence of SEQ ID NO: 284, the L2 comprises the amino acid sequence of SEQ ID NO: 286, and the L3 comprises the amino acid sequence of SEQ ID NO: 288. In yet other embodiments, the anti-IL33 antibody or antigen-binding fragment thereof comprises an HCVR comprising SEQ ID NO: 274 and an LCVR comprising SEQ ID NO: 282.

In some embodiments, the IL33 antibodies or antigen-binding fragments thereof comprise three heavy chain CDRs (HCDR1, HCDR2 and HCDR3) contained within a heavy chain variable region (HCVR) amino acid sequence selected from the group consisting of SEQ ID NO: 2, 18, 34, 50, 66, 82, 98, 114, 130, 146, 162, 178, 194, 210, 226, 242, 258, 274, 290, and 308; and comprises three light chain CDRs (LCDR1, LCDR2 and LCDR3) contained within a light chain variable region (LCVR) amino acid sequence selected from the group consisting of SEQ ID NOs: 10, 26, 42, 58, 74, 90, 106, 122, 138, 154, 170, 186, 202, 218, 234, 250, 266, 282, 298, and 316.

In some embodiments, the anti-IL33 antibodies, or antigen-binding fragments thereof comprise a HCVR and LCVR (HCVR/LCVR) sequence pair of SEQ ID NO: 2/10, 18/26, 34/42, 50/58, 66/74, 82/90, 98/106, 114/122, 130/138, 146/154, 162/170, 178/186, 194/202, 210/218, 226/234, 242/250, 258/266, 274/282, 290/298, or 308/316.

In some embodiments, the anti-IL33 antibodies, or antigen-binding fragments thereof comprise a heavy chain CDR1 (HCDR1) domain having an amino acid sequence selected from the group consisting of SEQ ID NO: 4, 20, 36, 52, 68, 84, 100, 116, 132, 148, 164, 180, 196, 212, 228, 244, 260, 276, 292, and 310, or a substantially similar sequence thereof having at least 90%, at least 95%, at least 98% or at least 99% sequence identity; a heavy chain CDR2 (HCDR2) domain having an amino acid sequence selected from the group consisting of SEQ ID NO: 6, 22, 38, 54, 70, 86, 102, 118, 134, 150, 166, 182, 198, 214, 230, 246, 262, 278, 294, and 312, or a substantially similar sequence thereof having at least 90%, at least 95%, at least 98% or at least 99% sequence identity; a light chain CDR1 (LCDR1) domain having an amino acid sequence selected from the group consisting of SEQ ID NO: 12, 28, 44, 60, 76, 92, 108, 124, 140, 156, 172, 188, 204, 220, 236, 252, 268, 284, 300, and 318, or a substantially similar sequence thereof having at least 90%, at least 95%, at least 98% or at least 99% sequence identity; and a light chain CDR2 (LCDR2) domain having an amino acid sequence selected from the group consisting of SEQ ID NO: 14, 30, 46, 62, 78, 94, 110, 126, 142, 158, 174, 190, 206, 222, 238, 254, 270, 286, 302, and 320, or a substantially similar sequence thereof having at least 90%, at least 95%, at least 98% or at least 99% sequence identity.

In some preferred embodiments, the anti-IL33 antibodies or antigen-binding fragments thereof comprise HCDR1-HCDR2-HCDR3-LCDR1-LCDR2-LCDR3 domains, respectively, having the amino acid sequences selected from the group consisting of: SEQ ID NOs: 4-6-8-12-14-16 (e.g. H1M9559N); 20-22-24-28-30-32 (e.g., H1M9566N); 36-38-40-44-46-48 (e.g., H1M9568N); 52-54-56-60-62-64 (e.g. H4H9629P); 68-70-72-76-78-80 (e.g., H4H9633P); 84-86-88-92-94-96 (e.g. H4H9640P); 100-102-104-108-110-112 (e.g., H4H9659P); 116-118-120-124-126-128 (e.g., H4H9660P); 132-134-136-140-142-144 (e.g., H4H9662P); 148-150-152-156-158-160 (e.g., H4H9663P); 164-166-168-172-174-176 (e.g., H4H9664P); 180-182-184-188-190-192 (e.g., H4H9665P); 196-198-200-204-206-208 (e.g., H4H9666P); 212-214-216-220-222-224 (e.g., H4H9667P); 228-230-232-236-238-240 (e.g., H4H9670P); 244-246-248-252-254-256 (e.g., H4H9671P); 260-262-264-268-270-272 (e.g., H4H9672P); 276-278-280-284-286-288 (e.g., H4H9675P); 292-294-296-300-302-304 (e.g., H4H9676P); and 310-312-314-318-320-322 (H1M9565N).

In some embodiments, the anti-IL33 antibodies, or antigen-binding fragments thereof, comprise the heavy and light chain CDR domains contained within heavy and light chain variable region (HCVR/LCVR) sequences of SEQ ID NO: 2/10, 18/26, 34/42, 50/58, 66/74, 82/90, 98/106, 114/122, 130/138, 146/154, 162/170, 178/186, 194/202, 210/218, 226/234, 242/250, 258/266, 274/282, 290/298, or308/316. The boundaries of CDRs may be according to the Kabat definition, the Chothia definition, or the AbM definition.

Other anti-IL33 antibodies and antigen-binding fragments thereof that may be used in the methods described herein are disclosed in European Publ. No. EP 1725261, PCT Publ. Nos. WO 2011/031600, WO 2015/099175, WO 2015/106080 (ANB020), WO 2016/077381, WO 2016/077366, or WO2016/156440, U.S. Pat. No. 8,187,596, and U.S. Publ. No. 2016/0168242.

In alternative preferred embodiments, an IL33 antagonist comprises an IL33 trap. IL33 traps comprise at least one IL33 binding domain, which comprises an IL33 binding portion of an IL33 receptor protein, designated ST2. In some embodiments, an IL33 trap further comprises an extracellular portion of an IL33 co-receptor, designated IL-1 receptor accessory protein, or IL-1RAcP. The IL33 trap may also comprise at least one multimerizing component, which functions to connect the various components of the trap with one another. The various components of the IL33 traps are described below and shown in Figure 1. IL33 traps may comprise any trap described in U.S. Publ. No. 2014/0271642 and PCT Publ. No. WO 2014/152195.

The IL33 trap may comprise a first IL33 binding domain (D1) attached to a multimerizing domain (M). In some embodiments, the IL33 trap comprises a second IL33 binding domain (D2) attached to D1 and/or M. In some preferred embodiments, D1 comprises an IL33-binding portion of an ST2 protein. In some preferred embodiments, D2 comprises an extracellular portion of an IL-1RAcP protein.

The individual components of the IL33 traps may be arranged relative to one another in a variety of ways that result in functional antagonist molecules capable of binding IL33. For example, D1 and/or D2 may be attached to the N-terminus of M. In some embodiments, D1 and/or D2 is attached to the C-terminus of M. In other embodiments, D1 is attached to the N-terminus of D2, and D2 is attached to the N-terminus of M, resulting in an in-line fusion, from N- to C-terminus, of an antagonist molecule represented by the formula D1-D2-M. Other orientations of the individual components are disclosed elsewhere herein in Figure 1.

The IL33 traps comprise at least one IL33 binding domain (sometimes referred to herein by the designation "D," or "D1," "D2," etc.). In some embodiments, the IL33 binding domain comprises an IL33-binding portion of an ST2 protein. An IL33-binding portion of an ST2 protein can comprise or consist of all or part of the extracellular domain of an ST2 protein. In preferred embodiments, an ST2 protein is a human ST2 protein, including the ST2 protein of amino acids 1-556 of accession number NP_057316.3 (SEQ ID NO: 352). In some alternative embodiments, the ST2 protein comprises an ST2 protein from a non-human species (e.g., mouse ST2, non-human primate ST2, etc.). An preferred IL33-binding portion of an ST2 protein is set forth herein as the amino acid sequence of SEQ ID NO: 328 (corresponding to the extracellular domain of human ST2 [K19-S328 of NCBI Accession No. NP_057316.3]). Other examples of an IL33-binding portion of an ST2 protein is set forth herein as the amino acid sequence of SEQ ID NO: 329 (corresponding to the extracellular domain of mouse ST2 [S27-R332 of NCBI Accession No. P14719]).

In some embodiments, the IL33 binding domain of the trap comprises an extracellular portion of an IL-1RAcP protein. In certain embodiments, an IL-1RAcP protein comprises a human IL-1RAcP protein, including an IL-1RAcP protein having the amino acid sequence of SEQ ID NO: 353. In some alternative embodiments, the IL-1RAcP protein comprises an IL-1RAcP protein from a non-human species (e.g., mouse IL-1RAcP, non-human primate IL-1RAcP, etc.). An exemplary extracellular portion of an IL-1RAcP protein is set forth herein as the amino acid sequence of SEQ ID NO: 330 (corresponding to the extracellular domain of human IL-1RAcP [S21-E359 of NCBI Accession No. Q9NPH3]). Another example of an extracellular portion of an IL-1RAcP protein is set forth herein as the amino acid sequence of SEQ ID NO: 331 (corresponding to the extracellular domain of mouse IL-1RAcP [S21-E359 of NCBI Accession No. Q61730]).

Non-limiting examples of IL33 traps for use in the methods are shown in Table 2, and include the IL33 traps designated "hST2-hFc," "hST2-mFc," "hST2-hIL1RAcP-mFc," "hST2-hIL1RAcP-hFc" and "mST2-mIL1RAcP-mFc." These correspond to SEQ ID NOs: 323, 324, 325, 326 and 327, respectively. IL33 receptor based traps may comprise an amino acid sequence that is at least about 80%, 85%, 90%, 91%, 92%, 93%, 94%, 95%, 96%, 97%, 98% or 99% identical to any of the exemplary IL33 receptor based traps set forth herein (e.g., SEQ ID NOs: 323, 324, 325, 326 and 327). IL33 traps may comprise D1 and/or D2 components having an amino acid sequence that is at least about 80%, 85%, 90%, 91%, 92%, 93%, 94%, 95%, 96%, 97%, 98% or 99% identical to any of the exemplary IL33 binding domain component amino acid sequences set forth herein (e.g., SEQ ID NOs: 328, 329, 330 and 331).

Five different exemplary IL33 traps were constructed. The first IL33 antagonist (hST2-hFc, SEQ ID NO: 323) includes the soluble extracellular region of human ST2 (SEQ ID NO: 328) fused at its C-terminus to the N-terminus of a human IgG1 Fc region (SEQ ID NO:332). The second IL33 antagonist (hST2-mFc, SEQ ID NO:324) consists of the soluble extracellular region of human ST2 (SEQ ID NO:328) fused at its C-terminus to the N-terminus of a mouse IgG2a Fc region (SEQ ID NO:333). The third IL33 antagonist (hST2-hIL1RAcP-mFc, SEQ ID NO: 325) consists of an in-line fusion having human ST2 (SEQ ID NO:328) at its N-terminus, followed by the extracellular region of human IL-1RAcP (SEQ ID NO:330), followed by a mouse IgG2a Fc (SEQ ID NO:333) at its C-terminus. The fourth IL33 antagonist (mST2-mIL1RAcP-mFc, SEQ ID NO: 326) consists of an in-line fusion having mouse ST2 (SEQ ID NO:329) at its N-terminus, followed by the extracellular region of mouse IL-1RAcP (SEQ ID NO:331), followed by a mouse IgG2a Fc (SEQ ID NO:333) at its C-terminus. The fifth IL33 antagonist (hST2-hIL1RAcP-hFc, SEQ ID NO:327) consists of an in line fusion having human ST2 of SEQ ID NO: 328 at its N-terminus, followed by the extracellular region of human IL-1RAcP (SEQ ID NO: 330) followed by a human IgG1 Fc (SEQ ID NO: 332) at its C terminus. See, Table 2.

**Table 2. Summary of IL33 Antagonists and the Component Parts**

| **IL33 Antagonist** | **Amino Acid Sequence of Full Antagonist Molecule** | **D1 Component** | **D2 Component** | **M Component** |
|---|---|---|---|---|
| hST2-hFc | SEQ ID NO:323 | human ST2 extracellular (SEQ ID NO:328) | Absent | human IgG1 Fc (SEQ ID NO:332) |
| hST2-mFc | SEQ ID NO:324 | human ST2 extracellular (SEQ ID NO:328) | Absent | mouse IgG2a Fc (SEQ ID NO:333) |
| hST2-hIL1RAcP-mFc | SEQ ID NO:325 | human ST2 extracellular (SEQ ID NO:328) | human IL-1RAcP extracellular (SEQ ID NO:330) | mouse IgG2a Fc (SEQ ID NO:333) |
| mST2-mIL1RAcP-mFc | SEQ ID NO:326 | mouse ST2 extracellular (SEQ ID NO:329) | mouse IL-1RAcP extracellular (SEQ ID NO:331) | mouse IgG2a Fc (SEQ ID NO:333) |
| hST2-hIL1RAcP-hFc | SEQ ID NO: 327 | human ST2 extracellular (SEQ ID NO:328) | human IL-1RAcP extracellular (SEQ ID NO:330) | human IgG1 Fc (SEQ ID NO:332) |

The IL33 traps may comprise at least one multimerizing domain (sometimes referred to herein by the abbreviation "M," "M1," "M2," etc.). In general terms, the multimerizing domain(s) function to connect the various components of the IL33 antagonists (e.g., the IL33-binding domain(s)) with one another. A multimerizing domain may comprise any macromolecule that has the ability to associate (covalently or non-covalently) with a second macromolecule of the same or similar structure or constitution. For example, a multimerizing domain may comprise a polypeptide comprising an immunoglobulin CH3 domain. A non-limiting example of a multimerizing domain is an Fc portion of an immunoglobulin, e.g., an Fc domain of an IgG selected from the isotypes IgG1, IgG2, IgG3, and IgG4, as well as any allotype within each isotype group.

Non-limiting exemplary multimerizing domains that can be used in the IL33 traps include human IgG1 Fc (SEQ ID NO: 332) or mouse IgG2a Fc (SEQ ID NO: 333). IL33 traps may comprise M components having an amino acid sequence that is at least about 80%, 85%, 90%, 91%, 92%, 93%, 94%, 95%, 96%, 97%, 98% or 99% identical to any of the exemplary M component amino acid sequences set forth herein (e.g., SEQ ID NOs: 332 or 333).

In some embodiments, the IL33 traps comprise two multimerizing domains, M1 and M2, wherein M1 and M2 are identical to one another. For example, M1 can be an Fc domain having a particular amino acid sequence, and M2 is an Fc domain with the same amino acid sequence as M1. The individual components of the IL33 antagonists (e.g., D1, D2, M, etc.) can be arranged relative to one another in a variety of ways. Non-limiting examples of all of the above noted arrangements, including an example of an IL33 trap comprising two multimerizing domains (M1 and M2) and four IL33 binding domains (D1, D2, D3 and D4,) are illustrated schematically in Figure 1.

The individual components of the IL33 traps (e.g., D1, D2, M1, M2, etc.) may be attached to one another directly (e.g., D1 and/or D2 may be directly attached to M, etc.); alternatively, the individual components may be attached to one another via a linker component (e.g., D1 and/or D2 may be attached to M via a linker oriented between the individual components; D1 may be attached to D2 via a linker; etc.).

Polypeptides that bind IL33 and/or its receptor (ST2 and/or IL-1 RAcP) and block ligand-receptor interaction are considered as IL33 antagonists and are disclosed in PCT Publ. No. WO 2014/152195. The biological characteristics of the IL33 traps are described in U.S. Publ. No. 2014/0271642.

Other agents that may act as IL33 antagonists and which may be used in the methods include immunoadhesins, peptibodies, and soluble ST2, or derivatives thereof; anti-IL33 receptor antibodies (e.g., anti-ST2 antibodies, for example, AMG-282 (Amgen) or STLM15 (Janssen) or any of the anti-ST2 antibodies described in PCT Publ. Nos. WO 2012/113813, WO 2013/173761, and WO 2013/165894, as well as U.S. Pat. Nos. 8,444,987 and 7,452,980. Other IL33 antagonists include ST2-Fc proteins, such as those described in PCT Publ. Nos. WO 2013/173761 and WO 2013/165894.

Without forming part of the invention, an IL-4R antagonist may be administered as part of a therapeutic regimen. The IL-4R antagonist is preferably administered in combination with the IL33 antagonist, though the IL-4R antagonist need not be administered at the same time as the IL33 antagonist. An IL-4R antagonist may comprise any agent that binds to or interacts with IL-4Rα or an IL-4R ligand, and inhibits or attenuates the normal biological signaling function of a type 1 and/or a type 2 IL-4 receptor. The IL-4R may comprise the amino acid sequence of SEQ ID NO: 347, or a biologically active fragment thereof. A type 1 IL-4 receptor is a dimeric receptor comprising an IL-4Rα chain and a γc chain. A type 2 IL-4 receptor is a dimeric receptor comprising an IL-4Rα chain and an IL-13Rα1 chain. Type 1 IL-4 receptors interact with and are stimulated by IL-4, while type 2 IL-4 receptors interact with and are stimulated by both IL-4 and IL-13. Thus, the IL-4R antagonists used in the methods may function by blocking IL-4-mediated signaling, IL-13-mediated signaling, or both IL-4- and IL-13-mediated signaling. The IL-4R antagonists may thus inhibit or prevent the interaction of IL-4 and/or IL-13 with a type 1 or type 2 receptor.

Non-limiting examples of categories of IL-4R antagonists include small molecule IL-4R antagonists, nucleic acid-based inhibitors of IL-4R expression or activity (e.g., siRNA or antisense), peptide-based molecules that specifically interact with IL-4R (e.g., peptibodies), "receptor-bodies" (e.g., engineered molecules comprising the ligand-binding domain of an IL-4R component), IL-4R-binding scaffold molecules (*e.g.*, DARPins, HEAT repeat proteins, ARM repeat proteins, tetratricopeptide repeat proteins, fibronectin-based scaffold constructs, and other scaffolds based on naturally occurring repeat proteins, and anti-IL-4R aptamers or portions thereof.

In preferred embodiments, an IL-4R antagonist comprises an antibody that specifically binds to human IL-4R. Antibodies are typically referred to herein according to the following nomenclature: Fc prefix (*e.g.* "H1M," or "H4H"), followed by a numerical identifier (*e.g.,* "9559," "9566," or "9629" as shown in Table 1), followed by a "P," or "N" suffix. According to this nomenclature, an antibody may be referred to herein as, e.g., "H1M9559N," "H1M9566N," "H4H9629P," etc. The H1M and H4H prefixes on the antibody designations used herein indicate the particular Fc region isotype of the antibody. For example, an "H1M" antibody has a mouse IgG1 Fc, whereas an "H4H" antibody has a human IgG4 Fc. An antibody having a particular Fc isotype can be converted to an antibody with a different Fc isotype (e.g., an antibody with a mouse IgG1 Fc can be converted to an antibody with a human IgG4, etc.), but in any event, the variable domains (including the CDRs) - which are indicated by the numerical identifiers shown in Table 1 - will remain the same, and the binding properties are expected to be identical or substantially similar regardless of the nature of the Fc

In preferred embodiments, the anti-IL-4R antibody is dupilumab. See U.S. Pat. Nos. 7,605,237, 7,608,693, and 9,290,574.

Human anti-IL-4R antibodies can be generated as described in U.S. Pat. No. 7,608,693. One exemplary IL-4R antibody is a mouse antibody specific for mouse IL-4R, and has the following amino acid sequences: a heavy chain variable region (HCVR) comprising SEQ ID NO: 335 and a light chain variable domain (LCVR) comprising SEQ ID NO: 336. The human anti-IL-4R antibody, referred to as dupilumab, specifically binds to human IL-4Rα and comprises a heavy chain variable region (HCVR) comprising SEQ ID NO: 337 and a light chain variable region (LCVR) comprising SEQ ID NO: 338, a heavy chain complementarity determining region 1 (HCDR1) comprising SEQ ID NO: 339, a HCDR2 comprising SEQ ID NO: 340, a HCDR3 comprising SEQ ID NO: 341, a light chain complementarity determining region 1 (LCDR1) comprising SEQ ID NO: 342, a LCDR2 comprising SEQ ID NO: 343 and a LCDR3 comprising SEQ ID NO: 344. The full-length heavy chain of dupilumab is shown as SEQ ID NO: 345 and the full length light chain is shown as SEQ ID NO: 346.

In some embodiments, the IL-4R antagonist comprises an anti-IL-4Rα antibody, or antigen-binding fragment thereof comprising a heavy chain variable region (HCVR), light chain variable region (LCVR), and/or complementarity determining regions (CDRs) comprising any of the amino acid sequences of the anti-IL-4R antibodies as set forth in U.S. Pat. Nos. 7,605,237 and 7,608,693. In some embodiments, the IL-4R antagonist comprises an anti-IL-4R antibody having the binding characteristics of the reference antibody referred to herein as dupilumab (U.S. Pat. No. 7,605,237 and U.S. Pat. No. 7,608,693). In some embodiments, the anti-IL-4Rα antibody or antigen-binding fragment thereof comprises the heavy chain complementarity determining regions (HCDRs) of a heavy chain variable region (HCVR) comprising the amino acid sequence of SEQ ID NO: 337 and the light chain complementarity determining regions (LCDRs) of a light chain variable region (LCVR) comprising the amino acid sequence of SEQ ID NO: 338. In some embodiments, the anti-IL-4Rα antibody or antigen-binding fragment thereof comprises three HCDRs (HCDR1, HCDR2 and HCDR3) and three LCDRs (LCDR1, LCDR2 and LCDR3), wherein the HCDR1 comprises the amino acid sequence of SEQ ID NO: 339; the HCDR2 comprises the amino acid sequence of SEQ ID NO: 340; the HCDR3 comprises the amino acid sequence of SEQ ID NO: 341; the LCDR1 comprises the amino acid sequence of SEQ ID NO: 342; the LCDR2 comprises the amino acid sequence of SEQ ID NO: 343; and the LCDR3 comprises the amino acid sequence of SEQ ID NO: 344. In yet other embodiments, the anti-IL-4R antibody or antigen-binding fragment thereof comprises an HCVR comprising SEQ ID NO: 337 and an LCVR comprising SEQ ID NO: 338. In yet other embodiments, the anti-IL-4R antibody or antigen-binding fragment thereof comprises an HCVR comprising SEQ ID NO: 335 and an LCVR comprising SEQ ID NO: 336. In some embodiments, the anti-IL-4R antibody or antigen-binding fragment thereof comprises a heavy chain (HC) amino acid sequence as set forth in SEQ ID NO: 345 and a light chain (LC) amino acid sequence as set forth in SEQ ID NO: 346.

In some embodiments, the IL-4R antibody or antigen-binding fragment thereof comprises the heavy chain complementarity determining regions (HCDRs) of a heavy chain variable region (HCVR) comprising the amino acid sequence of SEQ ID NO:335 or SEQ ID NO: 337 and the light chain complementarity determining regions (LCDRs) of a light chain variable region (LCVR) comprising the amino acid sequence of SEQ ID NO:336 or SEQ ID NO: 338.

In some embodiments, the IL-4R antibody or antigen-binding fragment thereof comprises three HCDRs (HCDR1, HCDR2 and HCDR3) and three LCDRs (LCDR1, LCDR2 and LCDR3), wherein the HCDR1 comprises the amino acid sequence of SEQ ID NO: 339, the HCDR2 comprises the amino acid sequence of SEQ ID NO:340; the HCDR3 comprises the amino acid sequence of SEQ ID NO:341; the LCDR1 comprises the amino acid sequence of SEQ ID NO:342; the LCDR2 comprises the amino acid sequence of SEQ ID NO:343; and the LCDR3 comprises the amino acid sequence of SEQ ID NO:344.

In some embodiments, the IL-4R antibody or antigen-binding fragment thereof for use in the methods of the disclosure comprises an HCVR comprising the amino acid sequence of SEQ ID NO: 335 or SEQ ID NO: 337 and an LCVR comprising the amino acid sequence of SEQ ID NO: 336 or SEQ ID NO: 338.

In some embodiments, the IL-4R antibody or antigen-binding fragment thereof for use in the methods of the disclosure comprises an HCVR/LCVR amino acid sequence pair of SEQ ID NOs: 335/336 or SEQ ID NOs: 337/338.

Other anti-IL-4Rα antibodies include, for example, the antibody referred to and known in the art as AMG317 (Corren et al., 2010, Am J Respir Crit Care Med., 181(8):788-796), or MEDI 9314, or any of the anti-IL-4Rα antibodies as set forth in any of U.S. Pat. Nos. 7,186,809, 7,605,237, 7,638,606, 8,092,804, 8,679,487, or 8,877,189.

The anti-IL-4Rα and the IL33 antibodies may have pH-dependent binding characteristics. For example, an anti-IL-4Rα antibody or an anti-IL33 antibody may exhibit reduced binding to IL-4Rα, or to IL33, respectively, at acidic pH as compared to neutral pH. Alternatively, an anti-IL-4Rα antibody or an anti-IL33 antibody may exhibit enhanced binding to its antigen at acidic pH as compared to neutral pH. An "acidic pH" includes pH values less than about 6.2, e.g., about 6.0, 5.95, 5.9, 5.85, 5.8, 5.75, 5.7, 5.65, 5.6, 5.55, 5.5, 5.45, 5.4, 5.35, 5.3, 5.25, 5.2, 5.15, 5.1, 5.05, 5.0, or less. A "neutral pH" includes a pH of about 7.0 to about 7.4, as well as about 7.0, 7.05, 7.1, 7.15, 7.2, 7.25, 7.3, 7.35, and 7.4.

In another aspect, an IL33 antagonist is used alone or in combination with an IL-4R antagonist for treating or inhibiting an inflammatory condition of the lungs. The IL33 antagonist or combination may be used for treating or inhibiting one or more of asthma, COPD, or ACOS. The IL33 antagonist or combination may be used for treating or inhibiting nasal polyps. The combination may be used for treating or inhibiting one or more of eosinophilic asthma, eosinophilic COPD, or eosinophilic ACOS. The IL33 antagonist or combination may be used for treating or inhibiting one or more of the high eosinophilic subset of eosinophilic asthma, eosinophilic COPD, or eosinophilic ACOS. The combination demonstrates enhanced efficacy, as compared to the treatment or inhibition obtained when each antibody is used alone as monotherapy.

In some embodiments, an IL33 antagonist or a combination of an IL33 antagonist and an IL-4R antagonist is used in the manufacture of a medicament for the treatment or inhibition of any one of eosinophilic asthma, eosinophilic Chronic Obstructive Pulmonary Disease (COPD), eosinophilic asthma- Chronic Obstructive Pulmonary Disease overlap syndrome (ACOS), high-eosinophil eosinophilic asthma, high-eosinophil eosinophilic COPD, high-eosinophil eosinophilic ACOS, or nasal polyps. In preferred embodiments, the IL33 antagonist or combination is used in the manufacture of a medicament for such treatment or inhibition of any one of eosinophilic asthma, eosinophilic COPD, eosinophilic ACOS, high-eosinophil eosinophilic asthma, high-eosinophil eosinophilic COPD, high-eosinophil eosinophilic ACOS, or nasal polyps when a patient thereof has one or more risk alleles associated with eosinophilic asthma, eosinophilic COPD, or eosinophilic ACOS in the intronic IL1RL1 variant rs1420101 (SEQ ID NO: 357) or variant in linkage disequilibrium thereof, in the IL33 variant rs1342326 (SEQ ID NO: 358) or variant in linkage disequilibrium thereof, or in both the intronic IL1RL1 variant rs1420101 (SEQ ID NO: 357) or variant in linkage disequilibrium thereof and the IL33 variant rs1342326 (SEQ ID NO: 358) or variant in linkage disequilibrium thereof.

According to such use, the IL33 antagonist may comprise an IL33 trap. The IL33 trap may comprise a first IL33 binding domain comprising an IL33 binding portion of IL1RL1 and a second IL33 binding domain comprising an extracellular portion of IL-1RAcP. The IL33 antagonist may alternatively comprise an antibody or antigen-binding fragment thereof that specifically binds to IL33. The antibody or antigen-binding fragment thereof that specifically binds to IL33 may comprise the H1, H2, and H3 domains of SEQ ID NO: 274 and the L1, L2, and L3 domains of SEQ ID NO: 282. The IL-4R antagonist may comprise an antibody or antigen-binding fragment thereof that specifically binds to IL-4R. The antibody or antigen-binding fragment thereof that specifically binds to IL-4R may comprise the H1, H2, and H3 domains of SEQ ID NO: 337 and the L1, L2, and L3 domains of SEQ ID NO: 338.

The following examples are provided to describe the disclosure in greater detail. They are intended to illustrate, not to limit, the disclosure.

### Example 1

### Anti-IL33 antibody, anti-IL-4R antibody, and a combination of both in a chronic House Dust Mite-induced fibrosis and severe lung inflammation model

Chronic inflammatory airway diseases are a consequence of recurrent episodes of airway inflammation predominantly due to repeated exposure to allergens or other pathogens. In humans, such chronic insults induce a vast array of pathologies that include pulmonary infiltration by immune cells, increased cytokine production, mucus production and collagen deposition. This increase in inflammatory cytokines and immune cell infiltrates, accompanied by intense airway remodeling leads to airway narrowing, hyperresponsiveness to inhaled triggers such as allergens or pathogens, airway obstruction and loss of lung function.

To determine the effect of anti-IL33 inhibition in a relevant *in vivo* model, a chronic house dust mite extract (HDM)-induced fibrosis and severe lung inflammation and remodeling study was conducted in mice that were homozygous for the expression of human IL33 in place of mouse IL33 (IL33 Humln mice). *See* U.S. Publ. Nos. 2015/0320021 and 2015/0320022. Chronic HDM extract exposure induces severe lung inflammation, resulting in significant cellular infiltrate, cytokine expression, and remodeling. Efficacy of an anti-IL33 antibody, an anti-mouse IL-4Rα antibody or a combination of both was compared in this model. The anti-mouse IL-4Rα antibody used in this study is designated M1M1875N and comprises the HCVR/LCVR amino acid sequence pair of SEQ ID NOs: 335/336. The anti-IL33 antibody used in this study is designated H4H9675P and comprises the HCVR/LCVR amino acid sequence pair of SEQ ID NOs: 274/282.

IL33 Humln mice were intranasally administered either 50µg house dust mite extract (HDM; Greer, #XPB70D3A2.5) diluted in 20µL of 1X phosphate buffered saline (PBS), or 20µL of 1X PBS for 3 days per week for 15 weeks. A second control group of IL33 HumIn mice were administered 50µg HDM extract diluted in 20µL of 1X PBS for 3 days per week for 11 weeks, to assess the severity of the disease at the onset of antibody treatment. Four groups of HDM challenged mice were injected subcutaneously with 25 mg/kg of either the anti-IL33 antibody H4H9675P, the anti-mouse IL-4Rα antibody M1M1875N, a combination of both antibodies, or an isotype control antibody starting after 11 weeks of HDM challenge and then twice per week until the end of the HDM challenge (4 weeks of antibody treatment). On day 108 of the study, all mice were sacrificed and their lungs were harvested. Experimental dosing and treatment protocol for groups of mice are shown in Table 3.

**Table 3. Experimental dosing and treatment protocol for groups of mice**

| **Group** | **Mice** | **Intranasal challenge** | **Length of intranasal challenge** | **Antibody** |
|---|---|---|---|---|
| 1 | IL33 Humln mice | 1X PBS | 15 weeks | None |
| 2 | IL33 Humln mice | 50µg HDM in 20µL 1X PBS | 11 weeks | None |
| 3 | IL33 Humln mice | 50µg HDM in 20µL 1X PBS | 15 weeks | None |
| 4 | IL33 Humln mice | 50µg HDM in 20µL 1X PBS | 15 weeks | Isotype control antibody |
| 5 | IL33 Humln mice | 50µg HDM in 20µL 1X PBS | 15 weeks | Anti-IL33 antibody (H4H9675P) |
| 6 | IL33 Humln mice | 50µg HDM in 20µL 1X PBS | 15 weeks | Anti-IL-4Rα antibody (M1M1875N) |
| 7 | IL33 Humln mice | 50µg HDM in 20µL 1X PBS | 15 weeks | Anti-IL33 (H4H9675P) antibody + Anti-IL-4Rα (M1M1875N) antibody |

**Lung harvest for cytokine analysis.** Elevated lung levels of key mediators such as the prototypic type 2 cytokines IL-4, IL-5, and IL-13, as well as cytokines more characteristic of type 1 immune responses, such as IL-1β or TNFα have been involved in human the development of lung diseases. Lung levels of these inflammatory cytokines were measured in the study.

After exsanguination, the cranial and middle lobes of the right lung from each mouse were removed and placed into tubes containing a solution of tissue protein extraction reagent (1X T-PER reagent; Pierce, #78510) supplemented with 1X Halt Protease inhibitor cocktail (Thermo Scientific, #87786). All further steps were performed on ice. The volume of T-PER Reagent (containing the protease inhibitor cocktail) was adjusted for each sample to match a 1:7 (w/v) tissue to T-PER ratio. Lung samples were mechanically disrupted using the TissueLyser II (Qiagen #85300). The resulting lysates were centrifuged to pellet debris. The supernatants containing the soluble protein extracts were transferred to fresh tubes and stored at 4°C until further analysis.

Total protein content in the lung protein extracts was measured using a Bradford assay. For the assay, 10µL of diluted extract samples were plated into 96 well plates in duplicates and mixed with 200µL of 1X Dye Reagent (Biorad, #500-0006). Serial dilutions of bovine serum albumin (BSA; Sigma, #A7979), starting at 700µg/mL in 1X T-Per reagent were used as a standard to determine the protein concentration of the extracts. After a 5-minute incubation at room temperature, absorbance at 595nm was measured on a Molecular Devices SPECTRAMAX^{®} M5 plate reader. Data analysis to determine total lung extract protein content based on the BSA standard was performed using GraphPad Prism^{™} software.

Cytokine concentrations in the lung protein extracts were measured using a Proinflammatory Panel 1 (mouse) multiplex immunoassay kit (MesoScale Discovery, # K15048G-2) and a custom mouse 6plex MULTI-SPOT^{®} immunoassay kit (MesoScale Discovery, #K152A41-4), according to the manufacturer's instructions. Briefly,50µL/well of calibrators and samples (diluted in Diluent 41) were added to plates pre-coated with capture antibodies and incubated at room temperature while shaking at 700 rpm for 2 hours. The plates were then washed 3 times with 1X PBS containing 0.05% (w/v) TWEEN^{®}-20 surfactant, followed by the addition of 25µL of Detection Antibody Solution diluted in Diluent 45. After a 2 hour incubation at room temperature while shaking, the plate was washed 3 times, and 150µL of 2X Read Buffer was added to each well. Electrochemiluminescence was immediately read on a MSD Spector instrument. Data analysis was performed using GraphPad Prism software.

Each cytokine concentration in lung total protein extracts from all mice in each group was normalized to the total protein content of the extracts measured by the Bradford assay, and expressed for each group as average pg of cytokine per mg of total lung proteins (pg/mg lung protein, ± SD) as shown in Table 4.

**Lung cytokines analysis.** As shown in Table 4, the level of the cytokines and chemokines IL-4, IL-5, IL-6, IL-1β and MCP-1 released in the lungs of IL33 HumIn mice receiving HDM for 15 weeks, with or without treatment with an isotype control antibody were significantly higher than in IL33 HumIn mice challenged with 1X PBS alone. Similarly, there was a trend towards an increased release of the cytokines IL-13 and TNFα in the lungs of IL33 HumIn mice receiving HDM for 15 weeks. In contrast, there was a significant reduction in the levels of IL-6, IL-13 and MCP-1 in the lungs of IL33 Humln mice treated with a combination of anti-IL33 and anti-mouse IL-4Rα antibodies during the last four weeks of the chronic HDM challenge as compared to IL33 HumIn mice administered HDM with an isotype control antibody during this time period. There was a trend towards reduced IL-4, IL-5, IL-1β and TNFα lung levels in IL33 Humln mice treated with a combination of anti-IL33 and anti-mouse IL-4Rα antibodies during the last four weeks of the chronic HDM challenge as compared to IL33 Humln mice administered HDM with an isotype control antibody during this time period. The effects on lung cytokines observed with the combination anti-IL33 and anti-mouse IL-4Rα antibodies was greater than treatment with either individual antibodies alone.

**Table 4. Cytokine concentration in lung protein extracts**

| Experi mental group | Mean [IL-4] in lung protein extracts (pg/mg lung protein) (±SD) | Mean [IL-5] in lung protein extracts (pg/mg lung protein) (±SD) | Mean [IL-13] in lung protein extracts (pg/mg lung protein) (±SD) | Mean [IL-6] in lung protein extracts (pg/mg lung protein) (±SD) | Mean [IL-1β] in lung protein extracts (pg/mg lung protein) (±SD) | Mean [TNFα] in lung protein extracts (pg/mg lung protein) (±SD) | Mean [MCP-1] in lung protein extracts (pg/mg lung protein) (±SD) |
|---|---|---|---|---|---|---|---|
| 1. 1X PBS challenge (n=5) | 0.13 (±0.17) | 0.80 (±1.41) | ND | 4.75 (±3.39) | 1.97 (±1.67) | 2.86 (±1.01) | 4.12 (±1.12) |
| 2. HDM challenge 11 weeks (n=4) | 5.71 (±3.76) * | 7.31 (±3.67) | 0.20 (±0.03) | 293.1 (±139.3) * | 181.8 (±131.0) * | 17.39 (±8.90) | 43.06 (±24.21) |
| 3. HDM challenge 15 weeks (n=4) | 2.70 (±1.71) | 5.13 (±3.20) | 0.19 (±0.03) | 308.3 (±390.1) | 51.79 (±16.97) | 15.38 (±8.11) | 105.6 (±106.5) * |
| 4. HDM challenge 15 weeks+ isotype control antibody (n=4) | 5.46 (±3.38) ** | 7.00 (±4.50) * | 0.22 (±0.02) | 395.0 (±270.1) ** | 162.3 (±166.5) ** | 19.57 (±14.81) | 141.7 (±126.3) ** |
| 5. HDM challenge 15 weeks + anti-IL33 antibody (n=5) | 1.15 (±1.38) | 1.93 (±1.90) | 0.20 (±0.02) | 136.8 (±164.1) | 122.9 (±194.1) | 17.05 (±4.48) * | 16.64 (±6.40) |
| 6. HDM challenge 15 weeks + anti-mouse IL-4Rα antibody (n=5) | 2.88 (±2.43) | 13.13 (±12.81) ** | 0.16 (±0.03) | 18.24 (±12.43) | 26.73 (±20.94) | 7.85 (±4.89) | 11.63 (±8.69) |
| 7. HDM challenge 15 weeks + anti-IL33 + anti-mouse IL-4Rα antibodies (n=5) | 0.47 (±0.13) | 0.73 (±0.37) | 0.10 (±0.05) ^{††} | 7.46 (±2.52) ^{†} | 3.722 (±1.59) | 3.07 (±1.34) | 4.62 (±1.27) ^{††} |

| | | | | | | | |
|---|---|---|---|---|---|---|---|
| Note: Statistical significance determined by Kruskal-Wallis One-way ANOVA with Dunn's multiple comparison post-hoc test is indicated (*= p<0.05, **= p<0.01, compared to groups 1: IL33 Humln mice, Saline challenge; ^{†}p<0.05, ^{††}p<0.01, compared to group 4: IL33 Humin mice, HDM challenge 15 weeks + Isotype control antibody). ND: Not determined. | | | | | | | |

**Lung harvest for gene expression analysis.** After exsanguination, the accessory lobe of the right lung from each mouse was removed, placed into tubes containing 400 µL of RNA Later (Ambion, #AM7020) and stored at -20°C until processing. Tissues were homogenized in TRIzol and chloroform was used for phase separation. The aqueous phase, containing total RNA, was purified using MagMAX^{™}-96 for Microarrays Total RNA Isolation Kit (Ambion by Life Technologies, #AM1839) according to manufacturer's specifications. Genomic DNA was removed using MagMAX^{™}Turbo^{™}DNase Buffer and TURBO DNase from the MagMAX kit listed above. mRNA (up to 2.5µg) was reverse-transcribed into cDNA using SuperScript^{®} VILO^{™} Master Mix (Invitrogen by Life Technologies, #11755500). cDNA was diluted to 2ng/ulL and 10ng cDNA was amplified with the TaqMan^{®} Gene Expression Master Mix (Applied Biosystems by Life Technologies, #4369542) and the relevant probes (Life Technologies; mouse *B2m:* Mm00437762_m1; mouse *114:* Mm00445259_m1; mouse *Il5:* Mm00439646_m1; mouse *Il13:* Mm00434204_m1; mouse *Il9:* Mm00434305_m1; mouse *116:* Mm00446190_m1; mouse *Ccl2:* Mm00441242_m1; mouse *Ccl11:* Mm00441238_m1 ; mouse *Ccl24:* Mm00444701_m1; mouse *Tnf:* Mm00443258_m1; mouse *Tgfb1:* Mm01178820_m1; mouse *Il1rl1:* Mm00516117_m1; mouse *Il13ra2*: Mm00515166_m1; mouse *Col15a1:* Mm00456584_m1; mouse *Col24a1:* Mm01323744_m1;) using the ABI 7900HT Sequence Detection System (Applied Biosystems). *B2m* was used as the internal control genes to normalize any cDNA input differences. The reference group used for normalization of all samples was the average of Group 1 samples ('1X PBS Challenge'). Expression of each gene was normalized to B2m expression within the same sample and expressed relative to its normalized expression in the reference group (mean ±SD), as shown in Table 5.

**Lung gene expression analysis.** As shown in Table 5, the level of expression of the cytokines, chemokines and collagen genes *114, Il13, 116, Ccl2, Tgfb1, Il13ra2* and *Col24a1* in the lungs of IL33 HumIn mice receiving HDM for 15 weeks, with or without treatment with an isotype control antibody were significantly increased compared to IL33 HumIn mice challenged with 1X PBS alone. Similarly, there was a trend towards an increase in expression of the genes *Il5, Il9, Ccl11, Ccl24, Tnf, Il1rl1* and *Col15a1* in the lungs of IL33 HumIn mice receiving HDM for 15 weeks.

In contrast, there was a significant reduction in the expression levels of *116, Ccl2, Ccl11* and *Ccl24* in the lungs of IL33 Humln mice treated with a combination of anti-IL33 and anti-mouse IL-4Rα antibodies during the last four weeks of the chronic HDM challenge as compared to IL33 Humln mice administered HDM with an isotype control antibody during this time period. There was a trend towards reduced *Il4, 115, Il13, Il19, Tnf, Tgfb1, Il1rl1, Il13ra2, Col15a1* and *Col24a1* expression levels in mice treated with a combination of anti-IL33 and anti-mouse IL-4Rα antibodies during the last four weeks of the chronic HDM challenge as compared to IL33 Humin mice administered HDM with an isotype control antibody during this time period. The effects on gene expression observed with the combination anti-IL33 and anti-mouse IL-4Rα antibodies was greater than treatment with either individual antibodies alone.

**Table 5. Gene expression (TaqMan) in mouse lungs.**

| Experi mental group | Mean Relative *Il4* expressi on in lung (±SD) | Mean Relative *Il5* expressi on in lung (±SD) | Mean Relative *Il13* expressi on in lung (±SD) | Mean Relative *Il9* expressi on in lung (±SD) | Mean Relative *Il6* expressi on in lung (±SD) | Mean Relative *Ccl2* expressi on in lung (±SD) | Mean Relative *Ccl11* expressi on in lung (±SD) | Mean Relative *Ccl24* expressi on in lung (±SD) |
|---|---|---|---|---|---|---|---|---|
| 1. 1X PBS challeng e (n=5) | 1.03 (±0.28) | 1.54 (±1.61) | 4.51 (±7.59) | 15.91 (±34.81) | 1.25 (±1.09) | 1.20 (±0.93) | 1.24 (±1.07) | 1.05 (±0.33) |
| 2. HDM challeng e 11 weeks (n=4) | 12.78 (±8.45) * | 7.13 (±3.49) | 114.1 (±68.3) * | 38.66 (±30.04) | 9.12 (±1.65) | 18.86 (±8.40) | 13.36 (±5.05) | 15.44 (±12.02) |
| 3. HDM challeng e 15 weeks (n=4) | 6.27 (±3.39) | 4.20 (±1.51) | 58.05 (±31.61) | 30.63 (±20.54) | 8.92 (±4.55) | 22.61 (±13.37) * | 8.65 (±3.20) | 4.58 (±1.91) |
| 4. HDM challeng e 15 weeks+ isotype control antibod y (n=4) | 10.98 (±5.46) * | 5.50 (±3.16) | 92.51 (±75.96) * | 19.51 (±10.29) | 13.80 (±6.98) ** | 24.53 (±9.13) ** | 12.14 (±7.82) | 12.41 (±8.73) |
| 5. HDM challeng e 15 weeks + anti-IL33 antibod y (n=5) | 2.80 (±3.11) | 1.74 (±1.11) | 12.91 (±12.93) | 0.00 (±0.00) | 3.87 (±3.00) | 5.20 (±2.44) | 6.21 (±3.55) | 1.45 (±2.09) |
| 6. HDM challeng e 15 weeks + anti-mouse IL-4Rα antibod y (n=5) | 1.87 (±1.03) | 7.98 (±6.52) | 69.56 (±66.86) * | 63.50 (±92.04) | 2.77 (±1.39) | 2.97 (±1.86) | 1.00 (±0.18) | 0.44 (±0.34) |
| 7. HDM challeng e 15 weeks + anti-IL33 + anti-mouse IL-4Rα antibodi es (n=5) | 1.37 (±0.35) | 1.56 (±0.97) | 9.34 (±3.10) | 0.57 (±1.27) | 1.04 (±0.31) ^{††} | **1.08** (±0.24) ^{††} § | 0.72 (±0.28) ^{†} | 0.15 (±0.10) ^{††} |

**Table 5 Continued: Gene expression (TaqMan) in mouse lungs.**

| Experimental group | Mean Relative *Tnf* expression in lung (±SD) | Mean Relative *Tgfb1* expression in lung (±SD) | Mean Relative *Il1rl1* expression in lung (±SD) | Mean Relative *Il13ra2* expression in lung (±SD) | Mean Relative *Col15a1* expression in lung (±SD) | Mean Relative *Col24a1* expression in lung (±SD) |
|---|---|---|---|---|---|---|
| 1. 1X PBS challenge (n=5) | 1.02 (±0.24) | 1.00 (±0.11) | 1.11 (±0.58) | 1.59 (±1.96) | 1.00 (±0.10) | 1.02 (±0.16) |
| 2. HDM challenge 11 weeks (n=4) | 1.45 (±0.41) | 1.40 (±0.27) | 3.03 (±0.88) * | 48.43 (±34.21) | 2.75 (±0.96) | 24.55 (±7.97) ** |
| 3. HDM challenge 15 weeks (n=4) | 1.58 (±0.43) | 1.32 (±0.33) | 2.53 (±0.79) * | 32.07 (±13.45) | 3.00 (±1.22) | 17.25 (±5.29) * |
| 4. HDM challenge 15 weeks+ isotype control antibody (n=4) | 1.59 (±0.78) | 1.37 (±0.12) * | 3.45 (±1.48) * | 52.02 (±40.63) | 3.80 (±0.96) * | 23.58 (±6.18) *** |
| 5. HDM challenge 15 weeks + anti-IL33 antibody (n=5) | 1.38 (±0.27) | 1.22 (±0.24) | 0.99 (±0.47) | 13.54 (±12.25) | 1.64 (±0.30) | 10.58 (±5.42) |
| 6. HDM challenge 15 weeks + anti-mouse IL-4Rα antibody (n=5) | 1.00 (±0.25) | 1.13 (±0.20) | 3.38 (±1.97) | 1.89 (±0.59) | 1.24 (±0.28) | 7.08 (±4.56) |
| 7. HDM challenge 15 weeks + anti-IL33 + anti-mouse IL-4Rα antibodies (n=5) | 0.68 (±0.08)^{§} | 1.09 (±0.12) | 1.12 (±0.57) | 1.89 (±0.27) | 0.74 (±0.21) ^{†† §} | 1.76 (±0.15) ^{†} |

| | | | | | | |
|---|---|---|---|---|---|---|
| Note: Statistical significance determined by Kruskal-Wallis One-way ANOVA with Dunn's multiple comparison post-hoc test is indicated (*= p<0.05, **= p<0.01, ***= p<0.01 compared to groups 1: IL33 HumIn mice, Saline challenge; ^{§}p<0.05, ^{§§}p<0.01, compared to group 3: IL33 Humin mice, HDM challenge 15 weeks; ^{†}p<0.05, ^{††}p<0.01, compared to group 4: IL33 Humin mice, HDM challenge 15 weeks + Isotype control antibody). | | | | | | |

**Lung harvest for pulmonary cell infiltrate analysis.** Pulmonary infiltration by immune cells is observed in multiple airway inflammatory diseases, including asthma and COPD. Neutrophilic lung inflammation has been associated with lower lung function and severe tissue remodeling in asthma patients. Eosinophilic lung inflammation is a hallmark of type 2 inflammation usually seen in atopic diseases. In humans, high CD4/CD8 ratios are observed in patients with granulomatous lung diseases and other chronic inflammatory conditions. Flow cytometry was used in the study to determine the level of cellular infiltration in the lungs of HDM-exposed mice.

After exsanguination, the caudal lobe of the right lung from each mouse was removed, chopped into cubes that were approximately 2 to 3 mm in size, and then placed into a tube containing a solution of 20 µg/mL DNAse (Roche, #10104159001) and 0.7 U/mL Liberase TH (Roche, #05401151001) diluted in Hank's Balanced Salt Solution (HBSS) (Gibco, #14025), which was incubated in a 37°C water bath for 20 minutes and vortexed every 5 minutes. The reaction was stopped by adding ethylenediaminetetraacetic acid (EDTA, Gibco, #15575) at a final concentration of 10mM. Each lung was subsequently dissociated using a gentleMACS dissociator (Miltenyi Biotec, #130-095-937), then filtered through a 70 µm filter and centrifuged. The resulting lung pellet was resuspended in 1 mL of 1X red blood cell lysing buffer (Sigma, #R7757) to remove red blood cells. After incubation for 3 minutes at room temperature, 3 mL of 1X DMEM was added to deactivate the red blood cell lysing buffer. The cell suspensions were then centrifuged, and the resulting cell pellets were resuspended in 5 mL of MACS buffer (autoMACS Running Buffer; Miltenyi Biotec, #130-091-221). The resuspended samples were filtered through a 70µm filter and 1x10⁶ cells per well were plated in a 96-well V-bottom plate. Cells were then centrifuged and the pellets were washed in 1X PBS. After a second centrifugation, the cell pellets were resuspended in 100µL of LIVE/DEAD Fixable Blue Dead Cell Stain (Life Technologies, # L23105) diluted at 1:500 in 1X PBS to determine cell viability and incubated for 20 minutes at room temperature while protected from light. After one wash in 1X PBS, cells were incubated in a solution of MACS buffer containing 10µg/mL of purified rat anti-mouse CD16/CD32 Fc Block, (Clone: 2.4G2; BD Biosciences, #553142) for 10 minutes at 4°C. The cells were then incubated in the appropriate 2x antibody mixture (described in Table 6) diluted in MACS buffer for 30 minutes at 4°C while protected from light. After antibody incubation, the cells were washed twice in MACS buffer, resuspended in BD CytoFix (BD Biosciences, #554655) and then incubated for 15 minutes at 4°C while protected from light. The cells were subsequently washed, resuspended in MACS buffer, and then transferred to BD FACS tubes (BD Biosciences, #352235) for analysis of cellular infiltrates by flow cytometry.

CD4 and CD8 T cells were defined as cells that were live, CD45⁺, SSC^{Lo}, FSC^{Lo}, CD3⁺, CD19⁻, CD4⁺, CD8⁻ and live, CD45⁺, SSC^{Lo}, FSC^{Lo}, CD3⁺, CD19⁻, CD4⁻, CD8⁺ respectively. Activated CD4 T cells were defined as cells that were live, CD45⁺, SSC^{Lo}, FSC^{Lo}, CD3⁺, CD19⁻, CD4⁺, CD8⁻, and CD69⁺. Activated CD8 T cells were defined as cells that were live, CD45⁺, SSC^{Lo}, FSC^{Lo}, CD3⁺, CD19⁻, CD4⁻, CD8⁺, and CD69⁺. Activated B cells were defined as cells that were live, CD45⁺, SSC^{Lo}, FSC^{Lo}, CD3⁻, CD19⁺, and CD69⁺. ST2+ CD4+ T cells were defined as cells that were live, CD45⁺, SSC^{Lo}, FSC^{Lo}, CD3+, CD19-, ST2+ and CD4⁺. Eosinophils were defined as live, CD45⁺, GR1⁻, CD11c^{lo}, SiglecF^{hi}. Alveolar macrophages were defined as live, CD45⁺, GR1⁻, CD11c^{Hi}, SiglecF^{hi}. Data for activated cells is expressed as frequency of activated cells (CD69⁺) within the parent population (CD4, ± SD). Data for ST2+ CD4+ T cells is expressed as frequency of T cells (defined as cells that were live, CD45⁺, SSC^{Lo}, FSC^{Lo}, CD3+ and CD19-). Data for Eosinophils and Alveolar macrophages are expressed as frequency of live cells. CD4/CD8 T cells ratio is calculated as the ratio of the frequency of CD4 T to the frequency of CD8 T cells within the live population. All data are shown in Table 7.

**Table 6. Antibodies Used for Flow Cytometry Analysis**

| **Antibody** | **Fluorochrome** | **Manufacturer** | **Catalogue Number** | **Final dilution** |
|---|---|---|---|---|
| CD45.2 | PerCP-Cy5.5 | eBioscience | 45-0454 | 1/800 |
| Siglec-F | BV 421 | BD | 562681 | 1/200 |
| F4/80 | APC | eBioscience | 17-4801-82 | 1/200 |
| Ly6G | BUV395 | BD | 563978 | 1/200 |
| Ly6C | PE-Cy7 | BD | 560593 | 1/100 |
| CD11c | PE | eBioscience | 12-0114-82 | 1/200 |
| CD11b | FITC | eBioscience | 53-0112-82 | 1/200 |
| CD19 | BV650 | BD | 562701 | 1/400 |
| CD3 | PE-Cy7 | BD | 552774 | 1/200 |
| CD4 | BV421 | BioLegend | 100438 | 1/200 |
| CD8 | BUV 395 | BD | 563786 | 1/400 |
| NKp46 (CD335) | FITC | eBioscience | 11-3351 | 1/800 |
| CD69 | PE | eBioscience | 12-0691 | 1/200 |
| CD25 | BV510 | BioLegend | 102042 | 1/200 |
| ST2 | APC | BioLegend | 145306 | 1/200 |

**Pulmonary cell infiltrate analysis.** As shown in Table 7, the frequency of eosinophils, activated B cells, activated CD8 cells, ST2+ Cd4+ T cells and CD4/CD8 T cells ratio in the lungs of IL33 Humln mice receiving HDM for 15 weeks, with or without treatment with an isotype control antibody were significantly higher than in IL33 Humln mice challenged with 1X PBS alone. Similarly, there was a trend towards an increased frequency of activated CD4 T cells in the lungs of IL33 Humln mice receiving HDM for 15 weeks. There was a trend towards a decreased frequency of alveolar macrophages detected by flow cytometry in the lungs of IL33 HumIn mice receiving HDM for 15 weeks, in the absence or presence of an isotype control antibody treatment. The frequency of alveolar macrophages was significantly increased in the lungs of IL33 HumIn mice treated with a combination of anti-IL33 and anti-mouse IL-4Rα antibodies during the last four weeks of the chronic HDM challenge as compared to IL33 HumIn mice administered HDM with an isotype control antibody during this time period. Similarly, there was a trend towards reduced frequency of eosinophils, activated CD4 and CD8 T cells, activated B cells, ST2+ CD4+ T cells as well as CD4/CD8 T cells ratio in the lungs of mice treated with a combination of anti-IL33 and anti-mouse IL-4Rα antibodies during the last four weeks of the chronic HDM challenge as compared to IL33 HumIn mice administered HDM with an isotype control antibody during this time period. The effects on frequency of eosinophils, alveolar macrophages, activated CD8 T cells, ST2+ CD4+ T cells and CD4/CD8 ratio in the lung observed for the combination anti-IL33 and anti-mouse IL-4Rα antibodies shows a trend towards greater efficacy than treatment with either individual antibodies alone.

**Table 7. Frequency of pulmonary cell infiltrate as determined by flow cytometry**

| Experimental group | Mean Frequency of Eosinophils in the live | Mean Frequency of Alveolar Macrophages in the live | Mean CD4/CD8 T cells ratio (±SD) | Mean Frequency of Activated cells in CD4 T cells | Mean Frequency of Activated cells in CD8 T cells | Mean Frequency of Activated cells in B cells | Mean Frequency of ST2+ CD4+ cells in T cells populatio |
|---|---|---|---|---|---|---|---|
| | population (±SD) | population (±SD) | | population (±SD) | population (±SD) | population (±SD) | n (±SD) |
| 1. 1X PBS challenge (n=5) | 1.45 (±0.92) | 5.05 (±1.64) | 3.00 (±1.48) | 13.12 (±9.89) | 3.26 (±1.64) | 0.39 (±1.17) | 3.25 (±4.15) |
| 2. HDM challenge 11 weeks (n=4) | 17.08 (±3.94) * | 2.34 (±0.93) | 6.42 (±2.71) | 49.95 (±8.76) | 9.58 (±7.44) | 4.67 (±1.47) ** | 32.60 (±12.23) |
| 3. HDM challenge 15 weeks (n=4) | 15.40 (±3.99) * | 4.92 (±1.55) | 6.95 (±0.71) ** | 58.53 (±5.76) | 15.68 (±3.03) * | 3.70 (±1.44) * | 37.33 (±8.98) * |
| 4. HDM challenge 15 weeks+ isotype control antibody (n=4) | 15.00 (±3.35) * | 2.33 (±1.60) | 7.49 (±1.28) * | 57.75 (±7.64) | 14.59 (±3.82) | 3.90 (±1.48) * | 37.96 (±16.71) * |
| 5. HDM challenge 15 weeks + anti-IL33 antibody (n=5) | 8.51 (±7.52) | 7.44 (±4.18) | 4.03 (±1.28) | 48.22 (±5.66) | 13.86 (±5.21) | 1.72 (±0.72) | 19.24 (±5.72) |
| 6. HDM challenge 15 weeks + anti-mouse IL-4Rα antibody (n=5) | 12.30 (±7.83) | 9.93 (±5.18) | 5.56 (±2.22) | 53.42 (±6.52) | 13.11 (±6.26) | 2.14 (±1.23) | 35.01 (±9.83) * |
| 7. HDM challenge 15 weeks + anti-IL33 + anti-mouse IL-4Rα antibodies (n=5) | 3.78 (±1.60) | 14.64 (±3.86) ^{†} | 2.96 (±0.93) | 42.52 (±9.79) | 7.90 (±1.30) | 1.74 (±0.91) | 11.78 (±3.73) |

| | | | | | | | |
|---|---|---|---|---|---|---|---|
| Note: Statistical significance determined by Kruskal-Wallis One-way ANOVA with Dunn's multiple comparison post-hoc test is indicated (*= p<0.05, **= p<0.01, compared to groups 1: IL33 HumIn mice, Saline challenge; ^{†}p<0.05, compared to group 4: IL33 Humin mice, HDM challenge 15 weeks + Isotype control antibody). | | | | | | | |

**Lung harvest for quantification of histopathology.** The inflammatory pattern observed in this model is accompanied by widespread and severe structural changes in HDM-exposed lungs, with evidence of goblet cell metaplasia, increases in sub-epithelial collagen deposition and significant pulmonary consolidation. These pathologies are known features of human inflammatory respiratory diseases that contribute to decline of lung function and airway hyperreactivity.

After exsanguination, the left lungs were removed and placed into plates containing a 3 mL solution of 4% (w/v) paraformaldehyde (Boston Bioproducts, # BM-155) in 1X phosphate buffered saline and stored at room temperature for 3 days. Lung samples were then blotted dry and transferred to tubes containing 70% ethanol for histological analysis. The samples were sent to Histoserv, Inc (Germantown, MD) for paraffin embedding, sectioning and periodic acid Schiff (PAS) or Hematoxylin and Eosin (H&E) staining.

**Quantification of Goblet cell metaplasia.** Goblet cell metaplasia and mucus hypersecretion are hallmarks of many pulmonary diseases including asthma, chronic obstructive pulmonary disease, and cystic fibrosis. Excessive mucus production leads to airway obstruction and affects several important outcomes such as lung function, health-related quality of life, exacerbations, hospitalizations, and mortality in humans. PAS-positive goblet cells and total epithelial cells were counted in a millimeter length of the primary bronchus. Goblet cell metaplasia is expressed as the frequency of PAS-positive cells in a millimeter of bronchial epithelium (%, ± SD) as shown in Table 8.

**Quantification of lung consolidation.** Lung consolidation includes the accumulation of solid or liquid material in the alveolar space. Lung consolidation is a compound endpoint likely reflecting the combination of cellular infiltrate, hyperplasia, and mucus production, used here as a measurement of gross pathology. The fraction of lung area occupied by the crystal bodies was quantified on Movat pentachrome stained paraffin-embedded lung sections using ImageJ software (NIH, Bethesda, MD). Using the particle analysis function, total lung area in the section, as well as consolidated area in the section were measured. The fraction of consolidated lung area is given by the ratio of both measurements, as shown in Table 8.

**Quantification of sub-epithelial fibrosis.** Sub-epithelial fibrosis includes an excess of interstitial collagen deposition beneath the pulmonary epithelium. Increased sub-epithelial fibrosis has been reported to be specifically associated with asthma in humans. In the model, sub-epithelial fibrosis was measured on Masson's trichrome stained paraffin-embedded lung sections using HaLo software (Indica Labs, NM). Using the Layer thickness tool, the thickness of the collagen layer beneath the bronchial epithelium was recorded multiple times, with about 30 µm intervals, across a millimeter of the primary bronchus. Sub-epithelial fibrosis is expressed as the mean thickness of the collagen layer beneath the epithelium (µm, ± SD) as shown in Table 8.

**Analysis of lung histopathology.** As shown in table 9, there was a trend towards an increase in goblet cell metaplasia in the lungs of IL33 Humln mice receiving HDM for 15 weeks, with or without treatment with an isotype control antibody compared to IL33 Humln mice challenged with 1X PBS alone. Similarly, there was a significant increase in lung consolidation, as well as in sub-epithelial collagen thickness, in IL33 HumIn mice receiving HDM for 15 weeks.

In contrast, there was trend towards a reduction in goblet cell metaplasia and sub-epithelial collagen thickness, and a significant reduction in lung consolidation in IL33 Humln mice treated with a combination of anti-IL33 and anti-mouse IL-4Rα antibodies during the last four weeks of the chronic HDM challenge as compared to IL33 HumIn mice administered HDM with an isotype control antibody during this time period. The effects on goblet cell metaplasia, lung consolidation and sub-epithelial collagen thickness observed for the combination anti-IL33 and anti-mouse IL-4Rα antibodies showed a trend towards greater efficacy than treatment with either individual antibodies alone.

**Table 8. Quantification of histopathology in mouse lungs**

| Experimental group | Mean Goblet cell metaplasia (% PAS-positive cells) (±SD) | Mean lung consolidation (% ±SD) | Mean sub-epithelial collagen thickness (µm) (±SD) |
|---|---|---|---|
| 1. 1X PBS challenge (n=5) | 32.94 (±43.61) | 6.97 (±3.72) | 25.90 (±4.00) |
| 2. HDM challenge 11 weeks (n=4) | 59.98 (±39.01) | 70.70 (±12.94) | 81.76 (±25.37) * |
| 3. HDM challenge 15 weeks (n=4) | 92.15 (±10.16) | 83.21 (±3.65) ** | 82.12 (±23.04) * |
| 4. HDM challenge 15 weeks+ isotype control antibody (n=4) | 81.60 (±17.56) | 84.16 (±5.85) ** | 63.11 (±11.87) |
| 5. HDM challenge 15 weeks + anti-IL33 antibody (n=5) | 39.22 (±18.93) | 58.82 (±18.26) | 70.99 (±23.85) |
| 6. HDM challenge 15 weeks + anti-mouse IL-4Rα antibody (n=5) | 79.82 (±25.02) | 57.79 (±18.72) | 57.62 (±15.34) |
| 7. HDM challenge 15 weeks + anti-IL33 + anti-mouse IL-4Rα antibodies (n=5) | 19.69 (±8.80) | 35.01 (±20.68) | 48.19 (±18.58) |

| | | | |
|---|---|---|---|
| Note: Statistical significance determined by Kruskal-Wallis One-way ANOVA with Dunn's multiple comparison post-hoc test is indicated (**= p<0.01, compared to groups 1: IL33 Humln mice, Saline challenge). | | | |

**Serum collection for IgE and HDM-specific IgG1 levels measurement.** To determine the total IgE concentration in the serum samples for each mouse, a sandwich ELISA OPTEIA kit (BD Biosciences, #555248) was used according to the manufacturer's instructions. Serum samples were diluted and incubated with anti-IgE capture antibody coated on 96-well plates. Total IgE was detected by biotinylated anti-mouse IgE secondary antibody. Purified horseradish peroxidase (HRP)-labeled mouse IgE was used as a standard. The chromagen 3,3',5,5'-tetramethylbenzidine (TMB) (BD OPTEIA substrate reagent set, BD, #555214) was used to detect HRP activity. A stop solution of 1 M sulfuric acid was then added, and absorbance at 450nm was measured on a Molecular Devices SpectraMax M5 plate reader. Data analysis was performed using Prism^{™} software. The mean amounts of circulating IgE levels in serum for each experimental group are expressed as ng/mL (± SD) as shown in Table 9.

To determine the HDM specific IgG1 levels in the serum samples from each mouse, an ELISA was utilized. HDM (Greer, #XPB70D3A2.5) coated plates were incubated with serially diluted mouse serum samples, followed by incubation with a rat anti-mouse IgG1-HRP conjugated antibody (BD Biosciences, # 559626). All samples were developed with a TMB solution and analyzed as described above. Relative levels of circulating IgG1 in serum were represented as titer units (titer units were calculated by multiplying the measured OD by a dilution factor required to achieve OD450 that was greater than two times background). The mean circulating HDM-specific IgG1 levels in serum for each experimental group are expressed as titer x 10⁶ (±SD) as shown in Table 9.

**Analysis of the circulation levels of IgE and HDM-specific IgG1.** As shown in Table 9, there was a significant increase in circulating levels of IgE in the serum of IL33 HumIn mice receiving HDM for 15 weeks, with or without treatment with an isotype control antibody in IL33 HumIn mice challenged with 1X PBS alone. Similarly, there was a trend towards an increased level of circulating HDM-specific IgG1 in the serum of IL33 HumIn mice receiving HDM for 15 weeks. In contrast, there was a significant decrease in circulating levels of IgE and a trend towards a decrease in circulating levels of HDM-specific IgG1 in the serum of IL33 Humln mice treated with a combination of anti-IL33 and anti-mouse IL-4Rα antibodies during the last four weeks of the chronic HDM challenge as compared to IL33 Humln mice administered HDM with an isotype control antibody.

**Table 9. Circulating levels of IgE and HDM-specific IgG1 in mouse serum.**

| Experimental group | Mean circulating IgE levels (µg/mL) (±SD) | Mean circulating HDM-specific IgG1 levels (Titer x 10⁶) (±SD) |
|---|---|---|
| 1. 1X PBS challenge (n=5) | 2.16 (±2.02) | ND |
| 2. HDM challenge 11 weeks (n=4) | 50.16 (±8.35) | 1.18 (±0.15) |
| 3. HDM challenge 15 weeks (n=4) | 131.38 (±106.84) * | 1.88 (±0.81) |
| 4. HDM challenge 15 weeks+ isotype control antibody (n=4) | 193.07 (±78.96) *** | 1.62 (±0.62) |
| 5. HDM challenge 15 weeks + anti-IL33 antibody (n=5) | 45.74 (±45.74) | 1.76 (±0.98) |
| 6. HDM challenge 15 weeks + anti-mouse IL-4Rα antibody (n=5) | 11.12 (±8.65) | 0.99 (±0.56) |
| 7. HDM challenge 15 weeks + anti-IL33 + anti-mouse IL-4Rα antibodies (n=5) | 6.45 (±5.79) ^{†} | 0.75 (±0.30) |

| | | |
|---|---|---|
| Note: Statistical significance determined by Kruskal-Wallis One-way ANOVA with Dunn's multiple comparison post-hoc test is indicated (*= p<0.05, **= p<0.01, ***= p<0.001, compared to groups 1: IL33 Humln mice, Saline challenge; ^{†}p<0.05, compared to group 4: IL33 Humin mice, HDM challenge 15 weeks + Isotype control antibody). ND: Not determined. | | |

A combination of H4H9675P and anti-miL-4Rα treatment initiated in the context of severe, mixed inflammation improves all inflammatory parameters measured, reducing most to baseline levels. Additionally, additive effects are observed on some of the most pernicious endpoints, including composite lung gross pathology, goblet cell metaplasia, lung cellular infiltration, and cytokine levels. Therefore, blocking both pathways simultaneously has the potential to impact multiple inflammatory mediators in the context of severe mixed inflammation and tissue pathology, and normalize multiple parameters to baseline.

### Example 2

### Genetic variants in IL33 and its receptor associate with both eosinophilic asthma and COPD

In this Example, the relationship between previously identified asthma risk variants at *IL33* and *IL1RL1* with risk of asthma, COPD, and ACOS was examined in the largest combined collection of such cases yet assembled, in which genetic data is linked to electronic health records. The importance of these variants to eosinophilic subtypes of asthma, COPD, and ACOS, as well as to related upper airway diseases such as nasal polyps was examined. In addition, the association between predicted loss-of-function variants (pLOF) in *IL1RL1* and *IL33* with these diseases was evaluated.

**Human Genetics Study Oversight.** The human genetics studies were conducted as part of the DiscovEHR study of the Regeneron Genetics Center (RGC) and the Geisinger Health System (GHS).

**DiscovEHR Participants and Disease Definitions.** At the time of this study, the DiscovEHR study comprised a total of 92,323 adult individuals enrolled in the MyCode^{®} Community Health Initiative of the GHS. For this study 86,004 and 83,339 individuals of European ancestry had phenotype, and exome sequencing and genotype data, respectively for analysis. Participants were recruited from outpatient primary care and specialty clinics. Eosinophil counts and disease diagnosis codes (the International Classification of Diseases, Ninth Revision [ICD-9]) were extracted from EHRs, which covered a median of 14 years of clinical care. Median EHR-documented eosinophil count measurements were derived from complete blood counts following removal of likely spurious values that were > 3 standard deviations from the intra-individual median value. Case status was assigned on the basis of ICD-9 codes if at least one of the following criteria were met: (1) a problem-list entry of the diagnosis code; or (2) an encounter diagnosis code entered for 2 separate clinical encounters on separate calendar days. Individuals were assigned one or more of the three case classifications (asthma, COPD and ACOS) based on ICD-9 diagnosis codes.

Control patients for all binary trait analyses were defined as individuals without a single ICD-9 diagnosis code of asthma or COPD.

**Sequencing and Genotyping.** Sample preparation and whole exome sequencing were performed. In brief, exome capture was performed using either NimbleGen probes (Roche, SeqCap VCRome) or Integrated DNA Technologies probes (IDT, xGEN Exome Research panel) with additional content according the respective manufacturer's recommended protocol. Captured DNA was PCR amplified and quantified by qRT-PCR (Kapa Biosystems). Multiplexed samples were sequenced using 75 bp paired-end sequencing on Illumina v4 HiSeq 2500 or HiSeq X sequencers to a coverage depth sufficient to provide greater than 20x haploid read depth of over 85% of targeted bases in 96% of samples (approximately 80x mean haploid read depth of targeted bases). Raw sequence data from each Illumina HiSeq 2500 run were uploaded to the DNAnexus platform for sequence read alignment and variant identification. Raw sequence data were converted from BCL files to sample-specific FASTQ-files, which were aligned to the human reference build GRCh38 with BWA-mem. Single nucleotide variants (SNV) and insertion/deletion (indel) sequence variants were identified using the Genome Analysis Toolkit. Samples with genotype rate less than 10% were excluded. For final analyses, exome data was available for 59,082 and 29,504 individuals of European Ancestry captured using VCRome xGEN probe sets, respectively.

Aliquots of DNA were genotyped using the Human OmniExpress Exome Beadchip or the Global Screening Array (Illumina Corp.). For final analyses, Chip data was available for 56,239 and 28,500 individuals of European ancestry assayed on the Omni and GSA BeadChips respectively

**Study Design and Statistical Analysis.** OMNI and GSA Chip data was used to evaluate two previously identified asthma risk variants *(IL33* (rs1342326) and *IL1RL1* (rs1420101))for association with obstructive lung diseases, other airway diseases, and circulating eosinophil counts. These variants were tested for association with disease under an additive model using logistic regression in PLINK or R, including age, age², sex, smoking status, and the first four principal components of ancestry as covariates. Median EHR-documented eosinophil counts were log-transformed and tested for association with genotypes under an additive genetic model using linear (PLINK, R) models controlling for the same covariates as above. All p-values correspond to additive genetic models. Resulting summary statistics from analyses on both platforms were combined by meta-analysis.

Under the same statistical framework, exome data was used to identify associations between pLOF variants aggregated within *IL1RL1* or *IL33* and obstructive lung disease outcomes and eosinophil counts. At each gene, individuals were coded 0 if they did not carry any pLOF, and 1 if they were heterozygous carriers of at least one pLOF; No homozygous pLOF carriers for either *IL1RL1* or *IL33* were observed in this study. Resulting summary statistics from analyses on both platforms were combined by meta-analysis.

A genetic risk score, reflecting the sum of risk alleles for two independent variants *(IL33* (rs1342326) and *IL1RL1* (rs1420101)), was also used as a predictor of obstructive lung disease outcomes and eosinophil counts using logistic and linear regression models and the same covariates described above. Individuals missing genotype data for either or both variants were excluded. The effects of carrying one, two, three, or four risk alleles were determined separately relative to individuals carrying no risk allele at either variant. Trends between increasing score and increasing eosinophil counts or disease risk were tested using the linear regression and the Cochran-Armitage test, respectively.

All statistical analyses were performed with the use of PLINK software (v1.90p) or R version 3.2.1.

Confirmation of previously identified asthma risk variants in **IL33** and **IL1RL1** with DiscovEHR eosinophil **counts** and **EHR-defined** asthma. Clinical characteristics of MyCode^{®} participants in the DiscovEHR study are described in Figure 8. Among 86,004 patients of European ancestry exome sequenced in this study, 13267 (15.4%) patients were diagnosed with asthma, 9783 (11.4%) patients with COPD, and 2993 (3.4%) patients with both asthma and COPD (referred to here as asthma-COPD overlap syndrome, or ACOS). Among 83,339 patients of European ancestry with available Chip data for this study, 12832 (15.4%) patients were diagnosed with asthma, 9536 (11.4%) patients with COPD, and 2909 (3.5%) patients with ACOS.

The first large GWAS of asthma identified an intronic *IL1RL1* variant (rs1420101) that was associated with both asthma and circulating eosinophil counts, and a subsequent GWAS identified an upstream *IL33* variant (rs1342326) that was associated with asthma. In this study, association of rs1420101 (*IL1RL1*) and rs1342326 *(IL33)* with asthma (meta allelic odds ratio (OR_{allelic}) was confirmed (95% confidence interval) 1.07 (1.04-1.11), *P*=8.2x10⁻⁷ and Meta-OR_{allelic} 1.09 (1.05-1.16), *P*=6.0x10⁻⁶, respectively) (Figure 3).

Additionally, both variants were associated with lifetime median circulating eosinophil counts (n=66,776 individuals) (Meta-beta = 0.0066 (0.0054 - 0.0079) eos/ml, *P*=2.0x10⁻²³ and Meta-beta = 0.0061 (0.0045 - 0.0078) eos/ml, *P*=2.0x10⁻¹³, respectively

**IL33 and IL1RL1 associations with asthma are specific to eosinophilic subset.** Eosinophilic asthma is recognized as an important subset of asthma, and seems to be associated with increased asthma severity and steroid refractoriness, as well as differential responsiveness to biologic therapies. Having confirmed the previously described associations between the *IL33* and *IL1RL1* variants and eosinophil counts as well as asthma, independently assessed as distinct phenotypes, the study next assessed whether these risks are connected through a specific association with the eosinophilic subset of asthma, and therefore associations in asthma patient subgroups stratified by high (>200 eos/µL) and low (≤200 eos/µL) median lifetime eosinophil counts were assessed (Figure 3). Both the *IL33* (rs1342326) and *IL1RL1* (rs1420101) variants were significantly associated only with the eosinophilic asthma subset (for *IL33,* the allelic meta odds ratio was 1.12 (1.06-1.18) in the high eosinophil group vs. 1.04 (0.98-1.09) in the low eosinophil group; for IL1RL1, the allelic odds ratio was 1.07 (1.04-1.1) in the high eosinophil group vs. 1.02 (0.98-1.06) in the low eosinophil group) (Figure 3).

**Novel associations between asthma risk variants in IL33 and IL1RL1 and increased risk of COPD and ACOS, specifically in eosinophilic subsets.** In addition to the above associations with eosinophilic asthma, it was further discovered that the *IL33* (rs1342326) and *IL1RL1* (rs1420101) variants are suggestively or marginally significantly associated with COPD (Figure 4, for *IL33,* Meta OR_{allelic}=1.04 (0.99-1.09), *P*=8.9x10⁻², and for *IL1RL1,* Meta-OR_{allelic}=1.04 (1-1.07), *P*=3.9x10⁻²) and ACOS (Figure 5 for *IL33,* Meta OR_{allelic} 1.08 (1.0-1.16), *P*=3.8x10⁻², and for *IL1RL1,* 1.06 (1.0-1.12), *P*=4.8x10⁻²).

As with asthma, eosinophilic subsets of COPD and ACOS are associated with more severe disease. To determine whether the *IL33* and *IL1RL1* associations with COPD and ACOS were also specific to eosinophilic subtypes, as we had seen in asthma, associations between *IL33* (rs1342326) and *IL1RL1* (rs1420101) in COPD and ACOS subgroups stratified by high (>200 eos/µL) and low (≤200 eos/µL) median lifetime eosinophil count were assessed (Figure 4 and 5). Both variants were suggestively associated with COPD and ACOS only in the disease subgroups characterized by high circulating eosinophils.

**Higher burden of risk-increasing alleles in the IL33 signaling pathway leads to larger increases in asthma, COPD and ACOS risk.** Since *IL33* and *IL1RL1* are part of the same signaling complex, and since these variants display notable allele-dosage dependence in their risk associations when analyzed individually, a two-variant genetic risk score was constructed by summing the number of risk alleles at *IL33* (rs1342326) and *IL1RL1* (rs1420101) (each individual had a score ranging from 0-4), and the association between the score and eosinophil counts and risk of asthma, COPD and ACOS was tested. Groups of individuals carrying each genetic risk score were compared to the group with zero risk alleles. In tests for trend, increasing genetic risk score was significantly associated with increasing eosinophil counts (Figure 2, *P*=1x10⁻³⁹) and increasing risk of asthma (Figure 6, P=3.27x10⁻¹²), COPD (Figure 6, *P*=6.65x10⁻³), and ACOS (*P*=4.3x10⁻³). The largest effects (exceeding nominal significance) were observed for patients carrying three risk alleles (Figure 2, for eosinophil counts, Meta beta=0.0071 (0.0057-0.0085) eos/ml, *P*=8.8x10⁻²⁴; for asthma (Figure 6), Meta OR=1.28 (1.17-1.41), *P*=6.99x10⁻⁸; for COPD (Figure 6), OR=1.17 (1.04-1.31), *P*=9.35x10⁻³); and for ACOS (Figure 6), OR=1.23 (1.03-1.48), *P*=2.49x10⁻² (Figure 2). Few individuals carried 4 risk alleles, and consequently effect size estimates had wide confidence intervals.

Associations between the two-variant score and high and low eosinophil patient subgroups was also assessed (Figure 7). In trend tests, the score was significantly associated with high-eosinophil subsets of asthma (*P*=1.37x10⁻¹⁵), COPD (*P*=3.9x10⁻⁸), and ACOS (*P*=1.8x10⁻⁵), but not with low-eosinophil subsets of asthma, COPD, or ACOS (P>0.05 for each disease). The largest score-specific effects were observed for patients carrying three risk alleles (for high eosinophil asthma, Meta-OR=1.61 (1.42-1.84), *P*=6.33x10⁻¹³; for high eosinophil COPD, Meta-OR=1.53 (1.31-1.79), *P*=4.76x10⁻⁸); and for high eosinophil ACOS, OR=1.7 (1.34-2.14), *P*=1x10⁻⁵. As previously noted, there were relatively few individuals carrying 4 risk alleles, and respective effect size estimates had wide confidence intervals.

Predicted loss-of-function (pLOF) variants in IL33 are associated with decreased circulating eosinophil counts and obstructive lung disease risk. In analyses of *IL33* pLOF variant rs146597587, IL33 inactivation is associated with reduced eosinophil counts (Meta-Beta = -0.02 (-0.03-0.0092,*P*=7.3x10⁻⁵) but not significantly with a reduced risk of eosinophilic asthma, COPD and ACOS (OR=0.82 (0.63-1.07), P=0.15, OR=0.99 (0.74-1.33), P=0.94,and OR=0.93 (0.56-1.53), P=0.76, respectively) (Figure 7). *IL1RL1* pLOF variation was not associated with risk of obstructive lung disease.

**Analysis of IL1RL1 variant rs1420101 and IL33 variant rs1342326 with other airway diseases.** The unified airway theory posits that asthma may co-occur with other airway disease due to common mechanisms. Therefore, other EHR-documented diseases of the airway for association with rs1420101 and rs1342326 were tested (Figure 9). The *IL33* variant rs1342326 and *IL1RL1* variant rs1420101 was associated with an increased risk of allergic rhinitis (Meta-OR 1.04 (1.01-1.08), *P* = 0.02; Meta-OR 1.04 (1.01-1.06), P=2.4x10⁻³) as well as an increased risk for nasal polyps (Meta-OR 1.48 (1.28-1.72),P = 1.2x10⁻⁷, Meta-OR 1.17 (1.04-1.33), P = 1.2x10⁻²). Furthermore, the burden of these common risk variants also significantly increased the risk for allergic rhinitis and nasal polys (P = 1.45x10⁻⁴, P =2.48x10⁻⁷). These results are consistent with a previous report implicating *IL33* common genetic variation in nasal polyps risk.

**Summary.** IL33 is thought to be involved in barrier defense in epithelial tissues, including the lung epithelium, and has been implicated in asthma pathogenesis. The two variants described in this Example, *IL33* (rs1342326) and *IL1RL1* (rs1420101), have been previously associated with asthma in several studies. These reproducible and independent associations (*IL33* is located on chromosome 9; *IL1RL1* is located on chromosome 2) with both the ligand (*IL33*) and its specific receptor (*IL1R1*) suggest a role for IL33 signaling in asthma risk.

The current study significantly extends those previous findings. By performing whole exome sequencing and genotyping in over 83,000 adult participants of the DiscovEHR study associations were confirmed between *IL33* and *IL1RL1* and eosinophil counts as well as asthma, independently assessed as distinct phenotypes. Furthermore, a suggestive association of the *IL33* and *IL1RL1* variants with increased risk of COPD and ACOS was demonstrated - providing a genetic link supporting possibility of a shared mechanistic etiology between all three of these highly prevalent lung diseases. Associations of these variants with nasal polyps and allergic rhinitis were also demonstrated. In addition, it was found that in individuals carrying a larger burden of these risk alleles across both loci, larger effects on disease risk were observed. Furthermore, heterozygous carriers of a rare pLOF variants in *IL33* had lower median lifetime eosinophil counts and trends reflecting about 20% decreased risk of asthma. It is believed that these data provide genetic evidence linking the IL33 pathway to asthma and possibly to COPD through an allelic series that includes both risk-increasing common alleles and risk-decreasing rare pLOF alleles.

It is believed that prior to this study, genetic variants in the IL33 pathway had not been previously associated with COPD. Similarly, it is believed that there was no prior genetic data linking the any pathway to the risk of the eosinophilic subsets of asthma, COPD and ACOS. The results of this Example suggest a link between enhanced IL33 signaling for increased risk of the eosinophilic subtypes of asthma and COPD and, the numerically higher risk associations seen with ACOS patients suggests that this entity at the intersection of these conditions may indeed have special features. In addition to providing a unifying genetic and mechanistic link between eosinophilic subsets of heretofore distinctly labeled obstructive lung diseases, the data also support the tenets of the "unified airway theory" that posits that eosinophilic lung diseases may represent a continuum with related upper airway diseases. In this respect, a markedly increased risk for the *IL33* variant in allergic rhinitis and nasal polyps was observed.

Although not statistically significant, the protective associations with *IL33* pLOF variants described in this Example are consistent with a recent study that demonstrated that a rare loss-of-function variant in *IL33* was protective in asthma, supporting the possibility that inhibition of IL33 signaling may be an important therapeutic strategy for obstructive lung diseases. The data, in particular, suggest a role for interleukin-33 blockade in the eosinophilic forms of obstructive lung diseases such as asthma and COPD, as well as for eosinophilic upper airway diseases such as allergic rhinitis and nasal polyps.

Relatedly, recent progress with biologics in the treatment of severe and steroid-resistant asthma seems to distinguish eosinophilic disease. Multiple therapies that target interleukin-5 and interleukin-13 seem to only benefit the eosinophilic subset of asthma patients, while an antibody (Dupilumab) that blocks both the interleukin-4 and interleukin-13 pathways has numerically greater benefits in the eosinophilic patients, but also seems to have profound activity in the low-eosinophil subset. These therapies also seem to have benefit in nasal polyps and allergic rhinitis. Consistent with these previously described differences in the responses of eosinophilic asthma patients to biologics therapies, the data from this Example suggests that interleukin-33 blockade might best target the eosinophilic subsets of asthma, ACOS and COPD.

Although this study has certain limitations, it nonetheless represents a real-world clinical care setting, and in this population *IL33* and *IL1RL1* genetic variation is associated with increased risk of diagnosis of both asthma and COPD. For the purposes of personalized treatment of patients, whether one arrives at a diagnostic label of asthma, COPD, or ACOS is perhaps less important than identifying the mechanistic pathology that is occurring in a particular patient or group of patients, and these data suggest that subsets of asthma, COPD and ACOS patients may in part be driven by excess IL33 activity. Mitigating against various limitations of these data is the remarkable consistency of the findings using genetic variants in two different genes within the same pathway - parallel results were seen for variants in the gene for IL33 as well as for its receptor. For variants in both genes, consistent risk associations were seen across multiple related EHR-defined disease settings, and within these disease settings, consistent results were also repeatedly noted specific to the eosinophilic subsets of these diseases. Another convincing aspect of the data involves the consistent and notable allele-dependence of most of the risk associations, as well as the added power resulting from the two-variant risk score analyses. Finally, the reciprocal findings with the *IL33* pLOF variants is also supportive.

These data suggest that genetic variation that enhances IL33 signaling contributes to increased risk of the eosinophilic forms of asthma, COPD and ACOS, and that pLOF genetic variants in IL33 may contribute to reduced risk of these diseases; risk of upper airway diseases such as nasal polyps also appears to be linked to IL33 signaling. Individuals carrying genetic variants that enhance IL33 signaling may represent an opportunity for precision medicine, as those particular asthma and COPD patients may benefit most from therapeutic blockade of IL33. The data also raise the possibility that patients suffering from eosinophilic airway disease, regardless of subtype and variant status, may benefit from inhibition of IL33.

## Claims

1. An IL33 antagonist for use in treating or inhibiting asthma-chronic obstructive pulmonary disease overlap syndrome (ACOS) in a subject having both the intronic IL1RL1 variant rs1420101 risk allele and the IL33 variant rs1342326 risk allele.

2. The IL33 antagonist for use according to claim 1, wherein the ACOS is eosinophilic ACOS.

3. The IL33 antagonist for use according to claim 1 or claim 2, wherein the IL33 antagonist comprises an IL33 trap.

4. The IL33 antagonist for use according to claim 1 or claim 2, wherein the IL33 antagonist comprises an antibody, or antigen-binding fragment thereof, that specifically binds to IL33 and comprises the complementary determining regions of a heavy chain comprising the amino acid sequence of SEQ ID NO:274 and the complementary determining regions of a light chain comprising the amino acid sequence of SEQ ID NO:282.

5. An IL-4R antagonist for use in treating or inhibiting asthma-chronic obstructive pulmonary disease overlap syndrome (ACOS) in a subject having both the intronic IL1RL1 variant rs1420101 risk allele and the IL33 variant rs1342326 risk allele.

6. The IL-4R antagonist for use according to claim 5, wherein the ACOS is eosinophilic ACOS.

7. The IL-4R antagonist for use according to claim 5 or claim 6, wherein the IL-4R antagonist comprises an antibody, or antigen-binding fragment thereof, that specifically binds to IL-4R.

8. The IL-4R antagonist for use according to claim 7, wherein the antibody or antigen-binding fragment thereof, that specifically binds to IL-4R comprises dupilumab.

9. A combination of an IL33 antagonist and an IL-4R antagonist for use in treating or inhibiting asthma-chronic obstructive pulmonary disease overlap syndrome (ACOS) in a subject having both the intronic IL1RL1 variant rs1420101 risk allele and the IL33 variant rs1342326 risk allele.

10. The combination of an IL33 antagonist and an IL-4R antagonist for use according to claim 9, wherein the ACOS is eosinophilic ACOS.

11. The combination of an IL33 antagonist and an IL-4R antagonist for use according to claim 9 or claim 10, wherein the IL33 antagonist comprises an IL33 trap.

12. The combination of an IL33 antagonist and an IL-4R antagonist for use according to claim 9 or claim 10, wherein the IL33 antagonist comprises an antibody, or antigen-binding fragment thereof, that specifically binds to IL33 and comprises the complementary determining regions of a heavy chain comprising the amino acid sequence of SEQ ID NO:274 and the complementary determining regions of a light chain comprising the amino acid sequence of SEQ ID NO:282.

13. The combination of an IL33 antagonist and an IL-4R antagonist for use according to claim 9 or claim 10, wherein the IL-4R antagonist comprises an antibody, or antigen-binding fragment thereof, that specifically binds to IL-4R.

14. The combination of an IL33 antagonist and an IL-4R antagonist for use according to claim 13, wherein the antibody or antigen-binding fragment thereof, that specifically binds to IL-4R comprises dupilumab.

## Patentansprüche

1. IL33-Antagonist zur Verwendung bei der Behandlung oder Hemmung des Asthma-Chronischobstruktive-Lungenerkrankung-Überlappungssyndroms (ACOS) bei einem Subjekt, das sowohl das Risikoallel der intronischen IL1RL1-Variante rs1420101 als auch das Risikoallel der IL33-Variante rs1342326 aufweist.

2. IL33-Antagonist zur Verwendung nach Anspruch 1, wobei das ACOS ein eosinophiles ACOS ist.

3. IL33-Antagonist zur Verwendung nach Anspruch 1 oder Anspruch 2, wobei der IL33-Antagonist eine IL33-Falle umfasst.

4. IL33-Antagonist zur Verwendung nach Anspruch 1 oder Anspruch 2, wobei der IL33-Antagonist einen Antikörper oder ein antigenbindendes Fragment davon umfasst, der bzw. das spezifisch an IL33 bindet und die komplementaritätsbestimmenden Regionen einer schweren Kette umfasst, die die Aminosäuresequenz von SEQ ID NO:274 aufweist, sowie die komplementaritätsbestimmenden Regionen einer leichten Kette umfasst, die die Aminosäuresequenz von SEQ ID NO:282 aufweist.

5. IL-4R-Antagonist zur Verwendung bei der Behandlung oder Hemmung des Asthma-Chronischobstruktive-Lungenerkrankung-Überlappungssyndroms (ACOS) bei einem Subjekt, das sowohl das Risikoallel der intronischen IL1RL1-Variante rs1420101 als auch das Risikoallel der IL33-Variante rs1342326 aufweist.

6. IL-4R-Antagonist zur Verwendung nach Anspruch 5, wobei das ACOS ein eosinophiles ACOS ist.

7. IL-4R-Antagonist zur Verwendung nach Anspruch 5 oder Anspruch 6, wobei der IL-4R-Antagonist einen Antikörper oder ein antigenbindendes Fragment davon umfasst, der bzw. das spezifisch an IL-4R bindet.

8. IL-4R-Antagonist zur Verwendung nach Anspruch 7, wobei der IL-4R-Antikörper Dupilumab umfasst.

9. Kombination aus einem IL33-Antagonisten und einem IL-4R-Antagonisten zur Verwendung bei der Behandlung oder Hemmung des Asthma-Chronisch-obstruktive-Lungenerkrankung-Überlappungssyndroms (ACOS) bei einem Subjekt, das sowohl das Risikoallel der intronischen IL1RL1-Variante rs1420101 als auch das Risikoallel der IL33-Variante rs1342326 aufweist.

10. Kombination aus einem IL33-Antagonisten und einem IL-4R-Antagonisten zur Verwendung nach Anspruch 9, wobei das ACOS ein eosinophiles ACOS ist.

11. Kombination aus einem IL33-Antagonisten und einem IL-4R-Antagonisten zur Verwendung nach Anspruch 9 oder Anspruch 10, wobei der IL33-Antagonist eine IL33-Falle umfasst.

12. Kombination aus einem IL33-Antagonisten und einem IL-4R-Antagonisten zur Verwendung nach Anspruch 9 oder Anspruch 10, wobei der IL33-Antagonist einen Antikörper oder ein antigenbindendes Fragment davon umfasst, der bzw. das spezifisch an IL33 bindet und die komplementaritätsbestimmenden Regionen einer schweren Kette umfasst, die die Aminosäuresequenz von SEQ ID NO:274 aufweist, sowie die komplementaritätsbestimmenden Regionen einer leichten Kette umfasst, die die Aminosäuresequenz von SEQ ID NO:282 aufweist.

13. Kombination aus einem IL33-Antagonisten und einem IL-4R-Antagonisten zur Verwendung nach Anspruch 9 oder Anspruch 10, wobei der IL-4R-Antagonist einen Antikörper oder ein antigenbindendes Fragment davon umfasst, der bzw. das spezifisch an IL-4R bindet.

14. Kombination aus einem IL33-Antagonisten und einem IL-4R-Antagonisten zur Verwendung nach Anspruch 13, wobei der IL-4R-Antikörper Dupilumab umfasst.

## Revendications

1. Antagoniste de l'IL33 pour utilisation dans le traitement ou l'inhibition du syndrome de chevauchement entre l'asthme et la maladie pulmonaire obstructive chronique (ACOS) chez un sujet présentant à la fois l'allèle de risque de la variante intronique IL1RL1 rs1420101 et l'allèle de risque de la variante IL33 rs1342326.

2. Antagoniste de l'IL33 pour utilisation selon la revendication 1, dans lequel l'ACOS est un ACOS éosinophilique.

3. Antagoniste de l'IL33 pour utilisation selon la revendication 1 ou la revendication 2, dans lequel l'antagoniste de l'IL33 comprend un piège à IL33.

4. Antagoniste de l'IL33 pour utilisation selon la revendication 1 ou la revendication 2, dans lequel l'antagoniste de l'IL33 comprend un anticorps, ou un fragment de liaison à l'antigène de celui-ci, qui se lie spécifiquement à l'IL33 et comprend les régions déterminant la complémentarité d'une chaîne lourde comprenant la séquence d'acides aminés de SEQ ID NO:274, ainsi que les régions déterminant la complémentarité d'une chaîne légère comprenant la séquence d'acides aminés de SEQ ID NO:282.

5. Antagoniste de l'IL-4R pour utilisation dans le traitement ou l'inhibition du syndrome de chevauchement entre l'asthme et la maladie pulmonaire obstructive chronique (ACOS) chez un sujet présentant à la fois l'allèle de risque de la variante intronique IL1RL1 rs1420101 et l'allèle de risque de la variante IL33 rs1342326.

6. Antagoniste de l'IL-4R pour utilisation selon la revendication 5, dans lequel l'ACOS est un ACOS éosinophilique.

7. Antagoniste de l'IL-4R pour utilisation selon la revendication 5 ou la revendication 6, dans lequel l'antagoniste de l'IL-4R comprend un anticorps, ou un fragment de liaison à l'antigène de celui-ci, qui se lie spécifiquement à l'IL-4R.

8. Antagoniste de l'IL-4R pour utilisation selon la revendication 7, dans lequel l'anticorps anti-IL-4R comprend le dupilumab.

9. Combinaison d'un antagoniste de l'IL33 et d'un antagoniste de l'IL-4R pour utilisation dans le traitement ou l'inhibition du syndrome de chevauchement entre l'asthme et la maladie pulmonaire obstructive chronique (ACOS) chez un sujet présentant à la fois l'allèle de risque de la variante intronique IL1RL1 rs1420101 et l'allèle de risque de la variante IL33 rs1342326.

10. Combinaison d'un antagoniste de l'IL33 et d'un antagoniste de l'IL-4R pour utilisation selon la revendication 9, dans lequel l'ACOS est un ACOS éosinophilique.

11. Combinaison d'un antagoniste de l'IL33 et d'un antagoniste de l'IL-4R pour utilisation selon la revendication 9 ou la revendication 10, dans lequel l'antagoniste de l'IL33 comprend un piège à IL33.

12. Combinaison d'un antagoniste de l'IL33 et d'un antagoniste de l'IL-4R pour utilisation selon la revendication 9 ou la revendication 10, dans lequel l'antagoniste de l'IL33 comprend un anticorps, ou un fragment de liaison à l'antigène de celui-ci, qui se lie spécifiquement à l'IL33 et comprend les régions déterminant la complémentarité d'une chaîne lourde comprenant la séquence d'acides aminés de SEQ ID NO:274, ainsi que les régions déterminant la complémentarité d'une chaîne légère comprenant la séquence d'acides aminés de SEQ ID NO:282.

13. Combinaison d'un antagoniste de l'IL33 et d'un antagoniste de l'IL-4R pour utilisation selon la revendication 9 ou la revendication 10, dans lequel l'antagoniste de l'IL-4R comprend un anticorps, ou un fragment de liaison à l'antigène de celui-ci, qui se lie spécifiquement à l'IL-4R.

14. Combinaison d'un antagoniste de l'IL33 et d'un antagoniste de l'IL-4R pour utilisation selon la revendication 13, dans lequel l'anticorps anti-IL-4R comprend le dupilumab.
